(19) Europäisches Patentamt
European Patent Office
Office européen des brevets

(11) Publication number: 0 300 692 A1

(12) **EUROPEAN PATENT APPLICATION**

(21) Application number: 88306478.4

(22) Date of filing: 15.07.88

(51) Int. Cl.⁴: C07D 231/06 , C07D 405/04 , C07D 405/12 , A01N 43/56 , //C07F7/18,C07C69/76

(30) Priority: 17.07.87 US 74795
03.06.88 US 199584

(43) Date of publication of application: 25.01.89 Bulletin 89/04

(84) Designated Contracting States: ES GR

(71) Applicant: E.I. DU PONT DE NEMOURS AND COMPANY
1007 Market Street
Wilmington Delaware 19898(US)

(72) Inventor: Lahm, George Philip
148 Fairhill Drive
Wilmington Delaware 19808(US)

(74) Representative: Hildyard, Edward Martin et al
Frank B. Dehn & Co. European Patent Attorneys Imperial House 15-19 Kingsway
London WC2B 6UZ(GB)

(54) **Insecticidal pyrazolines.**

(57) Pyrazoline derivatives of the formula:

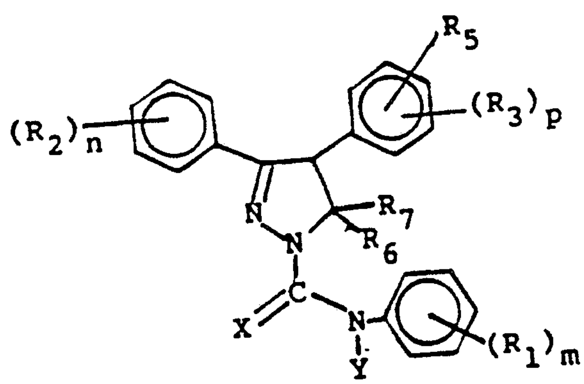

wherein $R_1$, $R_2$ and $R_3$ are independently selected from hydrogen or defined organic or inorganic substituents,
$R_5$ is a carboxylic acid, ester or amide substituent, or a sulfonamide group;
$R_6$ and $R_7$ are H or alkyl;
m, n and p are zero or integers;
X is O or S;
Y is H or an organic substituent;
exhibit activity against a wide spectrum of foliar and soil inhabiting insects, and hence may be used for crop protection.

EP 0 300 692 A1

## INSECTICIDAL PYRAZOLINES CROSS-REFERENCE TO RELATED APPLICATION

This application is a continuation-in-part of the application bearing U.S. Serial No. 74,795 filed on July 17, 1987.

## BACKGROUND OF THE INVENTION

The described triphenylpyrazolines and agricultural compositions containing them are useful as insecticides.

EPA 21,506 discloses insecticidal compounds of the formula

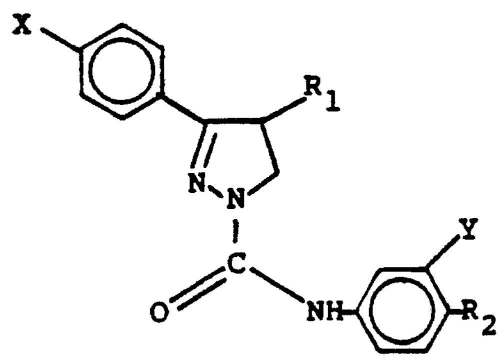

wherein
$R_1$ is a phenyl group;
$R_2$ is a halogenalkoxy, halogenalkenyloxy, halogenalkylthio, halogenalkenylthio, halogenalkylsulfonyl or halogenalkenylsulfonyl group having 1 to 6 carbon atoms;
and
X and Y represent hydrogen or halogen.

EPA 65,334 discloses insecticidal compounds of the formula

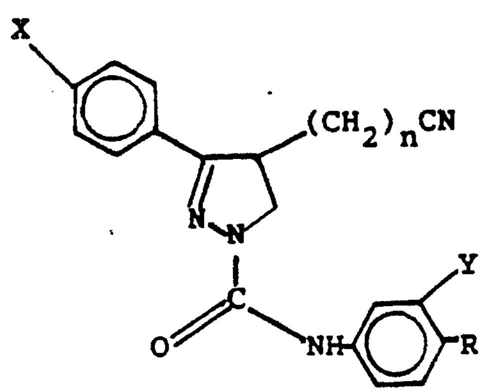

wherein
R is alkoxycarbonyl;
X and Y are hydrogen or halogen, and n is 2, 3, or 4.

EPA 58,424 discloses insecticidal compounds of the formula

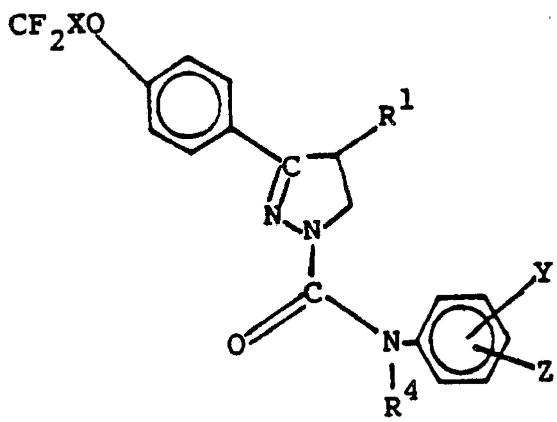

wherein
$R^1$ is phenyl;
$R^4$ is hydrogen or lower alkyl;
X is hydrogen or halogen;
Y and Z are hydrogen, halogen, lower alkyl, lower alkoxy, nitro, trifluoromethyl, lower alkylthio, acyl, nitrile, lower alkylsulfonyl, lower alkoxycarbonyl, A-$R^5$ or Y and Z form

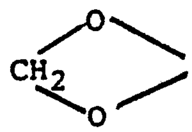 ;

A is O, S, SO, or $SO_2$ and $R^5$ is halogen-substituted lower alkyl.

EPA 113,213 discloses insecticidal compounds of the formula

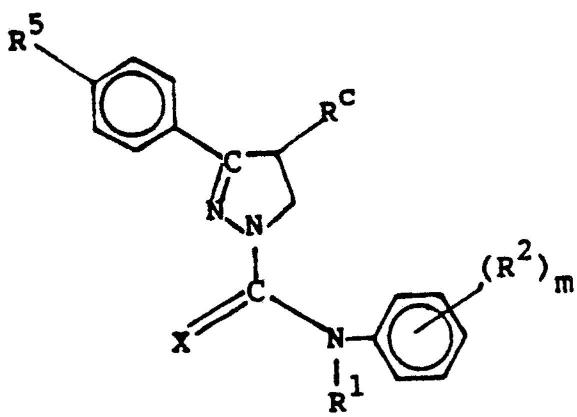

wherein
$R^1$ is hydrogen, alkyl or alkenyl;
X is oxygen or sulfur;
$R^2$ is halogen, alkyl, haloalkyl, cyano, nitro, alkoxy, haloalkoxy, alkylthio, haloalkylthio, alkylsulfonyloxy, alkylsulfonyl, alkoxycarbonyl or acyl, or two adjacent $R^2$ groups may form a ring;
m is 0 to 4;
$R^c$ is phenyl, para substituted by $R_4$;
$R^4$ and $R^5$ are $R^8SO_2O$, hydrogen, halogen, alkyl, haloalkoxy, alkylthio or alkylsulfonyl, wherein $R^8$ is alkyl or haloalkyl; at least one of $R^4$ and $R^5$ is $R^8SO_2O$, or $R^4$ is haloalkoxy.

EPA 153,127 discloses insecticidal compounds of the formula

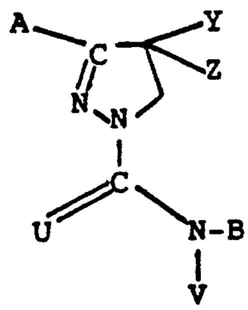

wherein
A and B are phenyl or substituted phenyl;
U is O or S;
V is hydrogen, cycloalkyl, aryl or $R^4$-Q;
Y is unsubstituted or substituted alkyl, unsubstituted or substituted aryl; and
Z is cycloalkyl, unsubstituted or substituted aryl or $R^4$-Q provided that Z is not hydrogen and Z is not methyl when Y is methyl; and $R^4$-Q is broadly defined.

U.S. Patent 4,156,007 discloses insecticidal compounds of the formula

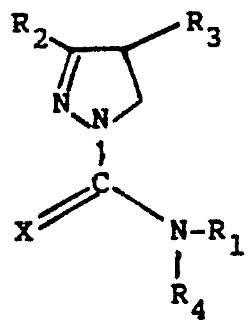

wherein
$R_1$ is phenyl or substituted phenyl;
$R_2$ and $R_3$ are equal or different and have the meanings of an alkyl group, a cycloalkyl group, a pyridyl or thienyl group which may be substituted with halogen, alkyl or nitro, a phenyl group or a phenyl group substituted with 1-2 substituents selected from the group consisting of a halogen atom, an alkyl group possibly substituted with halogen, a cycloalkyl group, an alkylthio group, an alkoxy group, a mono or dialkylamino group, a nitro group, a phenyl group possibly substituted with halogen, and a cyano group;
$R_4$ is hydrogen or alkyl; and
X is oxygen or sulfur.

## SUMMARY OF THE INVENTION

This invention pertains to compounds of Formula I, including all geometric and stereoisomers, agriculturally suitable salts thereof, agricultural compositions containing them and their use as insecticides:

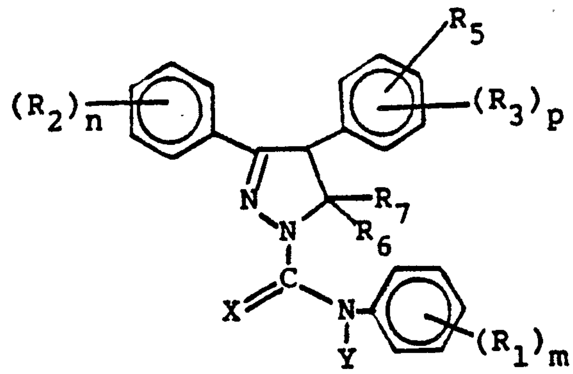

I

wherein

$R_1$, $R_2$ and $R_3$ are independently selected from $R_8$, halogen, CN, $N_3$, SCN, $NO_2$, $OR_8$, $SR_8$, $S(O)R_8$, $S(O)_2R_8$, $OC(O)R_8$, $OS(O)_2R_8$, $C(O)OR_8$, $C(O)R_8$, $C(O)NR_8R_9$, $S(O)_2NR_8R_9$, $NR_8R_9$, $NR_9C(O)R_8$, $OC(O)NHR_8$, $NR_9C(O)NHR_8$ and $NR_9S(O)_2R_8$, or when m, n or p is 2, $R_1$, $R_2$ or $R_3$ can be taken together as $OCH_2O$, $OCH_2CH_2O$ or $CH_2CH_2O$, to form a 5 or 6 membered ring, each of which can be substituted with 1 to 4 halogen atoms or 1 to 2 methyl groups;

$R_5$ is selected from $C(O)OR_{10}$, $C(O)R_{10}$, $C(O)NR_{10}R_{11}$, $C(S)SR_{10}$, $C(S)NR_{10}R_{11}$ and $S(O)_2NR_{10}R_{11}$;

$R_6$ is H or $C_1$ to $C_4$ alkyl;

$R_7$ is H or $CH_3$;

$R_8$ and $R_{10}$ are independently selected from H, $C_1$ to $C_4$ alkyl, $C_3$ to $C_4$ alkenyl, $C_3$ to $C_4$ alkynyl, $C_1$ to $C_4$ haloalkyl, $C_3$ to $C_4$ haloalkenyl, $C_1$ to $C_4$ alkyl substituted with CN, $C(O)OCH_3$, $C(O)OCH_2CH_3$, $OCH_3$, $OCH_2CH_3$, $SCH_3$, $SCH_2CH_3$ and $NO_2$, and phenyl or benzyl, either optionally substituted with W, or $R_8$ and $R_9$ can be taken together as $(CH_2)_4$, $(CH_2)_5$ or $CH_2CH_2OCH_2CH_2$;

$R_9$ and $R_{11}$ are independently selected from H, $C_1$ to $C_4$ alkyl, $C_3$ to $C_4$ alkenyl, $C_3$ to $C_4$ alkynyl,and $C_1$ to $C_4$ haloalkyl, or $R_{10}$ and $R_{11}$ can be taken together as $(CH_2)_4$, $(CH_2)_5$ or $CH_2CH_2OCH_2CH_2$;

m and n are independently 0 to 5;

p is 0 to 4;

W is selected from halogen, CN, $NO_2$, $C_1$ to $C_2$ alkyl, $C_1$ to $C_2$ haloalkyl, $C_1$ to $C_2$ alkoxy, $C_1$-$C_2$ haloalkoxy, $C_1$ to $C_2$ alkylthio, $C_1$ to $C_2$ haloalkylthio, $C_1$ to $C_2$ alkylsulfonyl or $C_1$ to $C_2$ haloalkylsulfonyl;

X is O or S; and

Y is selected from H, $C_1$ to $C_6$ alkyl, $C_2$ to $C_6$ alkoxyalkyl, CHO, $C_2$ to $C_6$ alkylcarbonyl, $C_2$ to $C_6$ alkoxycarbonyl, $C_2$ to $C_6$ haloalkylcarbonyl, $C_1$ to $C_6$ alkylthio, $C_1$ to $C_6$ haloalkylthio, phenylthio, and phenylthio substituted with 1 to 3 substituents independently selected from W.

Preferred compounds A are those of Formula I wherein:

$R_6$ is H;

$R_7$ is H;

n and p are independently 0 to 2; and

m is 1 to 2.

Preferred compounds B are preferred compounds A wherein:

$R_1$, $R_2$, and $R_3$ are independently $R_8$, halogen, CN, $NO_2$, $OR_8$, $SR_8$, $S(O)R_8$, $S(O)_2R_8$ or $NR_8R_9$, or when m, n or p is 2, $R_1$, $R_2$ or $R_3$ can be taken together as $OCH_2O$, $OCH_2CH_2O$ or $CH_2CH_2O$, each of which can be substituted with 1 to 4 halogen atoms or 1 to 2 methyl groups;

$R_8$ is $C_1$ to $C_2$ alkyl, $C_3$ to $C_4$ alkenyl, $C_1$ to $C_2$ haloalkyl, $C_3$ to $C_4$ haloalkenyl or phenyl optionally substituted with halogen;

$R_9$ is H or $C_1$ to $C_2$ alkyl; and

X is O.

Preferred compounds C are preferred compounds B wherein:

$R_1$ is halogen, CN, $NO_2$, $OCF_2H$, $OCF_3$ $OCF_2CF_2H$, $CF_3$ or when m is 2 then $R_1$ may be taken together as $CH_2C(CH_3)_2O$ or $CF_2CF_2O$ to form a 5 membered ring.

$R_2$ is H, halogen, CN, $NO_2$, $OCH_3$, $OCF_2H$, $OCF_3$, $SCH_3$, $SCF_2H$, $SCF_3$, $CF_3$, $OCF_2CF_2H$ or phenoxy;

$R_3$ is halogen;
$R_5$ is $C(O)OR_{10}$, $C(O)R_{10}$, $C(O)NR_{10}R_{11}$ or $S(O)_2NR_{10}R_{11}$;
$R_{10}$ is H, $C_1$ to $C_4$ alkyl, $C_3$ to $C_4$ alkenyl, $C_3$ to $C_4$ alkynyl, $C_1$ to $C_4$ haloalkyl, $C_3$ to $C_4$ haloalkenyl, $C_1$ to $C_4$ alkyl substituted with CN, $C(O)OCH_3$, $C(O)OCH_2CH_3$, $OCH_3$, $OCH_2CH_3$, $SCH_3$, $SCH_2CH_3$ and $NO_2$;
$R_{11}$ is H or $C_1$ to $C_2$ alkyl; and
Y is H, $C_1$ to $C_6$ alkyl, CHO, $C_2$ to $C_6$ alkylcarbonyl or $C_2$ to $C_6$ alkoxycarbonyl.

Preferred compounds D are preferred compounds C wherein:
$R_5$ is $C(O)OR_{10}$;
$R_{10}$ is $C_1$ to $C_3$ alkyl, $C_2$ to $C_3$ haloalkyl, propargyl or allyl; and one of $R_3$ or $R_5$ is in the para-position and one of $R_1$ is in the para-position; and
Y is H, $CH_3$ $C(O)CH_3$, $C(O)OCH_3$ or CHO.

Specifically preferred compounds are E to L:

E. A compound of Preferred D which is methyl 4-[3-(4-chlorophenyl)-4,5-dihydro-1-[[4-(trifluoromethyl)phenylamino]carbonyl]-1H-pyrazol-4-yl]benzoate;

F. A compound of Preferred D which is methyl 4-[3-(4-chlorophenyl)-1-[(4-chlorophenyl) aminocarbonyl]-4,5-dihydro-1H-pyrazol-4-yl]benzoate;

G. A compound of Preferred D which is methyl 4-[3-(4-fluorophenyl)-4,5-dihydro-1-[[4-(trifluoromethyl)phenylamino]carbonyl]-1H-pyrazol-4-yl]benzoate;

H. A compound of Preferred D which is methyl 4-[1-(4-chlorophenyl)aminocarbonyl]-[3-(4-fluorophenyl)-4,5-dihydro-1H-pyrazol-4-yl] benzoate;

I. A compound of Preferred D which is ethyl 4-[3-(4-chlorophenyl)-4,5-dihydro-1-[[4-(trifluoromethyl)-phenylamino]carbonyl]-1H-pyrazol-4-yl]benzoate;

J. A compound of Preferred D which is ethyl 4-[1-[(4-bromophenyl)aminocarbonyl]-3-(4-chlorophenyl)-4,5-dihydro-1H-pyrazol-4-yl] benzoate;

K. A compound of Preferred D which is methyl, 4-[3-(4-bromophenyl)-4,5-dihydro-1-[[4-(trifluoromethyl)phenylamino]carbonyl]-1H-pyrazol-4-yl]benzoate; and

L. A compound of Preferred D which is methyl 4-[3-(4-cyanophenyl)-4,5-dihydro-1-[[4-(trifluoromethyl)phenylamino]carbonyl]-1H-pyrazol-4-yl]benzoate.

Hereafter for the sake of brevity, the compounds of this invention are described and claimed as "triphenyl pyrazoline(s)" or "pyrazoline(s)" or "compound(s)", it being understood that such terms include all geometric and stereoisomers and all agriculturally suitable salts of said compounds.

## DETAILS OF THE INVENTION

Compounds of Formula I can be prepared from deoxybenzoins of Formula II by a three-step process whereby the Formula II compound is condensed with an aldehyde or ketone, then cyclized with hydrazine and finally reacted with a suitably substituted aryl isocyanate to yield the Formula I compound (Scheme I). Examples of this procedure, where $R_6$ and $R_7$ are equal to hydrogen, can be found in U.S. 4,070,365.

An alternative procedure, where $R_6$ and $R_7$ is other than hydrogen, involves first alkylation of the Formula II compound with an alkyl halide followed by bromination/dehydrobromination to yield the $\alpha,\beta$-unsaturated ketone IIA. Subsequent reactions with hydrazine and an aryl isocyanate yield the compounds of Formula I (Scheme IA). Examples of this procedure are described in European Patent Application 4,733.

Scheme I

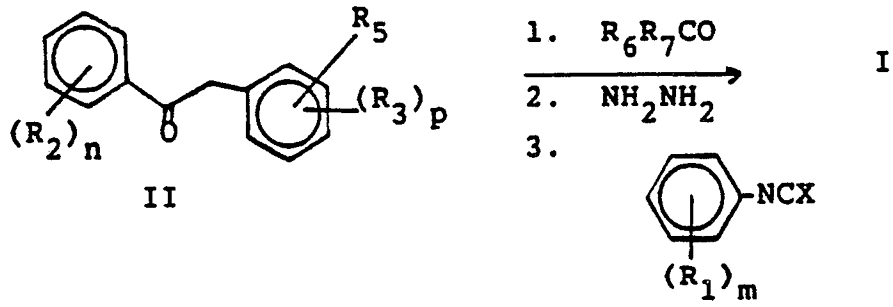


Scheme IA

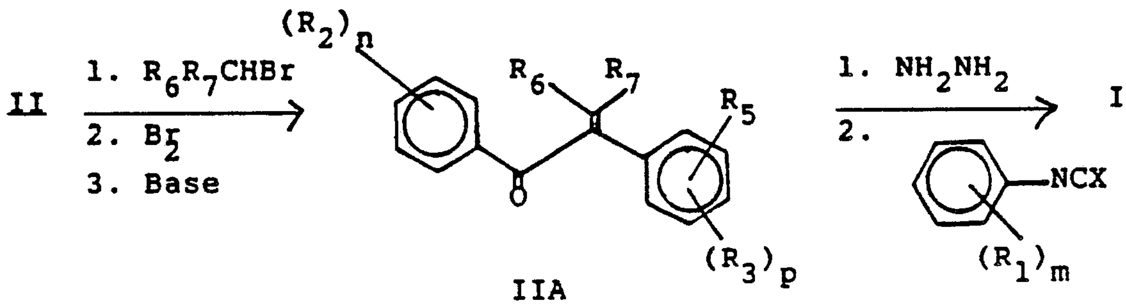


The intermediate deoxybenzoin of Formula II can be prepared by various procedures known in the art. The particular method chosen will depend on the substituents $R_2$, $R_3$, and $R_5$ and their compatibility with the conditions and reagents of that method. For example, compounds of Formula II, where $R_5$ is alkoxycarbonyl or dialkylsulfamoyl, can be prepared by the alkylation of a trimethylsilyl cyanohydrin of Formula III with a benzyl halide of Formula IV followed by conversion of the alkylated trimethylsilyl cyanohydrin to the corresponding carbonyl. The alkylation is typically run in a solvent such as ether or tetrahydrofuran at temperatures in the range of -78° to 25° C. Deprotonation of the Formula III compound is accomplished by treatment with a strong base such as lithium diisopropylamide and the Formula IV compound is subsequently added. Once alkylation is complete, the trimethylsilyl cyanohydrin of Formula V is converted to the deoxybenzoin of Formula II by procedures documented in the art. One method involves treatment of the Formula V compound with fluoride resulting in direct formation of the compounds of Formula II. Alternatively, the Formula II compounds can be prepared from the intermediates of Formula V by acid catalyzed cleavage of the trimethylsilyl group followed by base catalyed regeneration of the carbonyl. These procedures are depicted in Scheme II.

## Scheme II

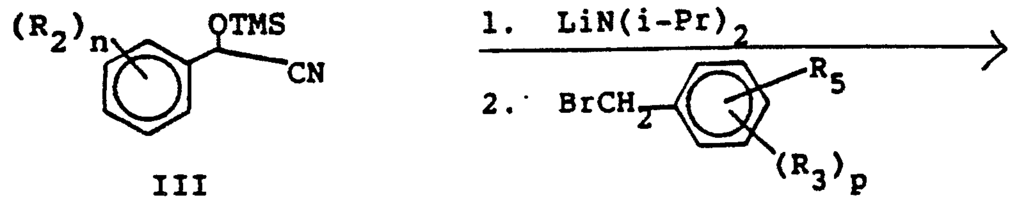


III IV

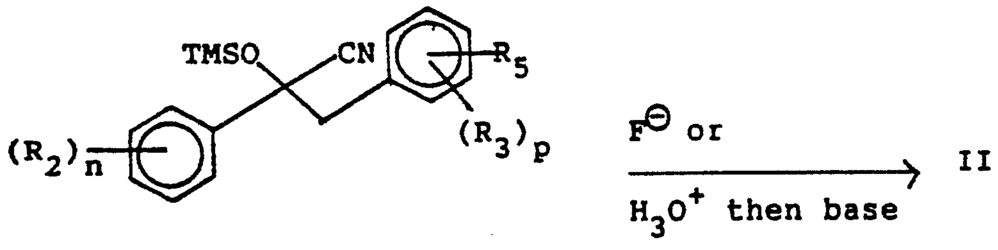


V

Alternatively, compounds of Formula II are accessible via a Friedel-Crafts acylation as depicted in Scheme III. This procedure is particularly useful for the preparation of a variety of $R_5$ substituents including $C(O)_2R_{10}$, $C(O)NR_{10}R_{11}$ and $SO_2NR_{10}R_{11}$. Generally, best results are achieved when $R_2$ is an ortho-para directing group such as alkyl, halogen, alkoxy and the like.

## Scheme III

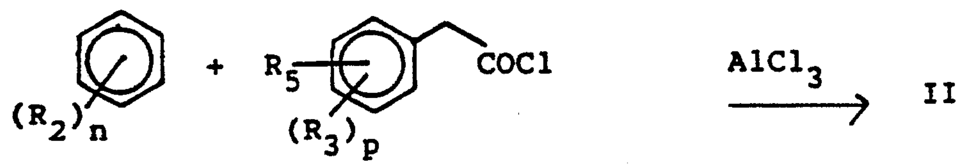


VI VII

Compounds of Formula II where $R_5$ is $C(O)R_{10}$ can be prepared as outlined in Scheme IV from the corresponding bromo derivative IX by metal-halogen exchange followed by reaction with an electrophilic C-(O)$R_{10}$ reagent. Dialkylamides (e.g., dimethylformamide, dimethylacetamide) are particularly suitable reagents for this transformation. Removal of the carbonyl protecting group affords the Formula II compound.

Scheme IV

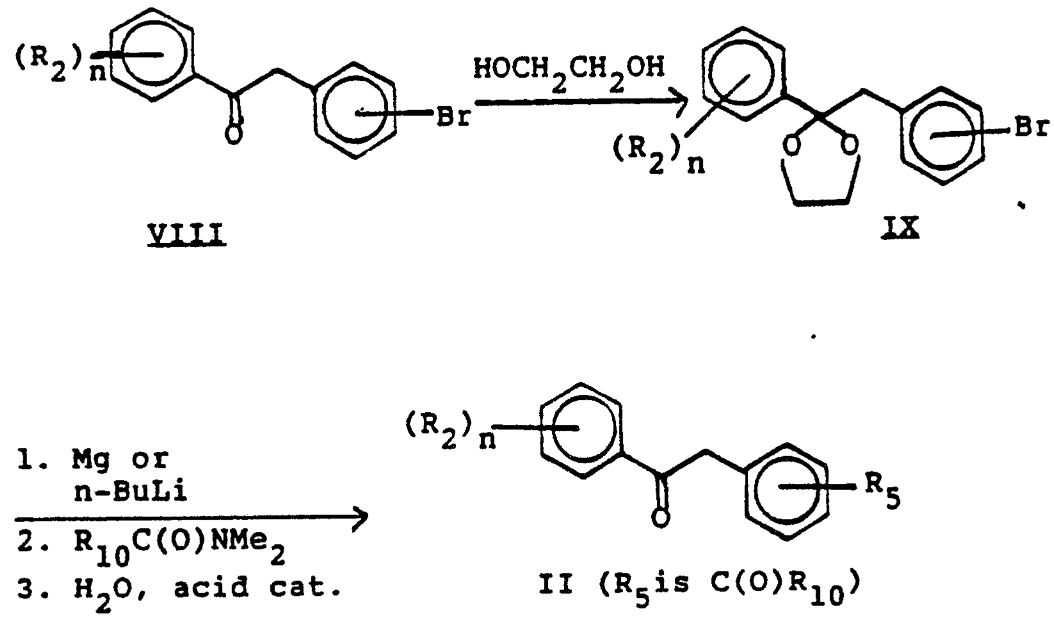


VIII

IX

II ($R_5$ is $C(O)R_{10}$)

Trimethylsilyl cyanohydrins of Formula III can be prepared from the corresponding benzaldehyde derivatives by reaction with trimethylsilylnitrile in the presence of a Lewis acid catalyst such as zinc iodide; see Evans et al., J. Chem. Soc. Chem. Comm., 55 (1973).

The following Examples illustrate preparation of the compounds of this invention.

## Example 1

### Step A

4-Chloro-α-(trimethylsilyloxy)benzeneacetonitrile

To a mixture of 32 g of 4-chlorobenzaldehyde and 0.4 g zinc iodide in 40 ml of methylene chloride was added 25 g of trimethylsilylnitrile dropwise under nitrogen. The addition was initiated at room temperature and added at such a rate that gentle reflux of the methylene chloride was maintained. After 24 hours, the reaction was concentrated and the residual oil distilled at 1.0 mm (bath temperature 150 °C) using a Kugelrohr apparatus to afford 50.74 g of the title compound as a yellow oil.

$^1$H NMR ($CDCl_3$)
δ 0.24 (s, 9H);
5.50 (s, 1H);
7.41 (s, 4H).

### Step B

Methyl 4-(bromomethyl)benzoate

To a solution of 10 g of methyl 4-hydroxymethylbenzoate and 80 g of carbon tetrabromide in 300 ml of diethyl ether was added 63.2 g of triphenylphosphine. The reaction was warmed to room temperature overnight, filtered through Celite® and concentrated to 87.2 g of a yellow oil.
Chromatography on silica gel (95:5, hexane:ethyl acetate) afforded 19.62 g of a white solid, m.p. 52° to 55°C.
$^1$H NMR ($CDCl_3$)
δ 3.92 (s, 3H)
4.50 (s, 2H);
7.45 (d, 2H);
8.01 (d, 2H)

Step C

Methyl 4- 2-(4-chlorophenyl)-2-oxoethyl benzoate

To a -70°C solution of 2.0 ml of diisopropylamine in 20 ml of THF, under nitrogen, was added 5.1 ml of 2.5 M n-butyllithium and the mixture was stirred for 5 min. A solution of 3.0 g of 4-chloro-α-(trimethylsilyloxy)benzeneacetonitrile in 5 to 10 ml of THF was then added dropwise such that the reaction was maintained at less than -60°C. Once added the reaction was stirred for 15 min, and then a solution of methyl 4-(bromomethyl)benzoate in 5 to 10 ml of THF was added dropwise and the reaction was then gradually warmed to room temperature. After 24 hours, 40 ml of 5% aqueous sodium bicarbonate was added and the reaction was concentrated. The residue was dissolved in 50 ml of methanol and 4.0 g of potassium fluoride was added. After stirring for 4 hours, the reaction was concentrated and the residue was partitioned between chloroform and water. The chloroform extracts were dried over magnesium sulfate and concentrated to 3.6 g of an orange solid. Trituration with n-butyl chloride afforded 2.5 g of a white solid, m.p. 165° to 167°C.
$^1$H NMR ($CDCl_3$)
δ 3.90 (s, 3H);
4.32 (s, 2H);
7.32 (d, 2H);
7.44 (d, 2H);
7.97 (d, 2H);
8.01 (d, 2H).
IR (Nujol) 1680, 1710 $cm^{-1}$.

Step D

Methyl 4-[3-(4-chlorophenyl)-4,5-dihydro-1H-pyrazol-4-yl] benzoate

A mixture of 2.0 g of the title ester of Step C, 3.0 ml of 37% formaldehyde, 0.2 ml of piperidine and 0.2 ml of glacial acetic acid was heated at reflux, under nitrogen, for 2 hours after which time TLC indicated the reaction was complete. The mixture was cooled to room temperature, partitioned between $CHCl_3$ and 5% aqueous $NaHCO_3$, dried over magnesium sulfate, filtered and concentrated to afford 2.5 g of a yellow oil. The residual oil was taken up in 10 ml of methanol and 0.35 ml of hydrazine hydrate was added. After heating at reflux for 2 hours, a white solid began to precipitate. The reaction was cooled to room temperature, filtered and dried to afford 0.76 g of a pale yellow powder, m.p. 155° to 159°C. $^1$H NMR ($CDCl_3$)
δ 3.58 (dd, 1H);
3.89 (s, 3H);
4.02 (dd, 1H);

4.55 (dd, 1H);
7.1-7.3 (m, 4H);
7.47 (d, 2H);
7.97 (d, 2H).

Step E

Methyl 4-[3-(4-chlorophenyl)-4,5-dihydro-1-[[4-(trifluoromethyl)-phenylamino]carbonyl-1H-pyrazol-4-yl]-benzoate

A mixture of 0.31 g of the title ester of Step D and 0.19 g of 4-trifluoromethylphenylisocyanate in 8 ml of 1:1 ether-THF was stirred 24 hours at room temperature and then concentrated. Trituration of the residue with ether/hexane afforded 0.28 g of the title compound as a yellow solid, m.p. 168° to 171 °C. $^1H$ NMR ($CDCl_3$)
δ 3.90 (s, 3H);
4.10 (dd, 1H);
4.45 (t, 1H);
4.82 (dd, 1H);
7.2-8.1 (m, 12H);
8.1 (bs, 1H).

By the general procedures described therein, or obvious modifications thereof, the compounds of Tables 1 to 3 can be prepared.

General Structure for Tables 1 to 3

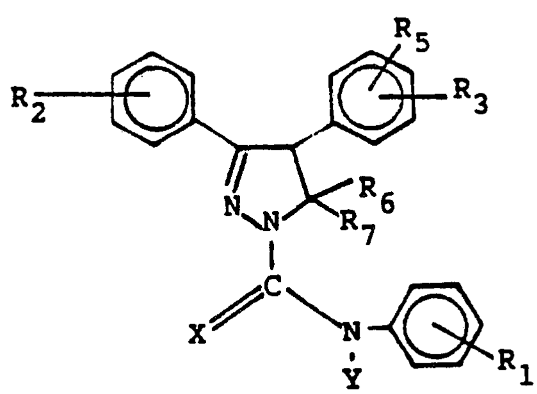

Table

1 X is O, $R_6$ is H, $R_7$ is H
2 X is S, $R_6$ is H, $R_7$ is H
3 X is O, Y is H

The following numbering system describes the compounds of Tables 1 to 3.

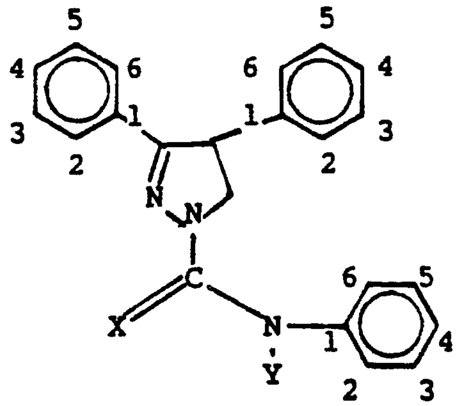

Table 1

| $R_1$ | $R_2$ | $R_3$ | $R_5$ | Y | Phys. Prop. |
|---|---|---|---|---|---|
| 4-$CF_3$ | H | H | 4-$CO_2$Me | H | m.p. 149 to 150.5°C |
| 4-Cl | H | H | 4-$CO_2$Me | H | m.p. 168 to 170°C |
| 4-Br | H | H | 4-$CO_2$Me | H | m.p. 172 to 174°C |
| 4-$OCF_2$H | H | H | 4-$CO_2$Me | H | |
| 4-F | H | H | 4-$CO_2$Me | H | |
| 4-$OCF_3$ | H | H | 4-$CO_2$Me | H | |
| 4-$CF_3$-3-Cl | H | H | 4-$CO_2$Me | H | |
| 4-$SCF_2$H | H | H | 4-$CO_2$Me | H | |
| 4-$NO_2$ | H | H | 4-$CO_2$Me | H | |
| 4-$CO_2$Me | H | H | 4-$CO_2$Me | H | |
| 4-OMe | H | H | 4-$CO_2$Me | H | |
| 3,4-$OCF_2CF_2$ | H | H | 4-$CO_2$Me | H | |
| 3,4-di-Cl | H | H | 4-$CO_2$Me | H | |
| 4-Cl | 4-Cl | H | 4-$CO_2$Me | H | m.p. 132 to 133°C |
| 4-Br | 4-Cl | H | 4-$CO_2$Me | H | m.p. 140 to 145°C |
| 4-$OCF_2$H | 4-Cl | H | 4-$CO_2$Me | H | |
| 4-F | 4-Cl | H | 4-$CO_2$Me | H | |
| 4-$OCF_3$ | 4-Cl | H | 4-$CO_2$Me | H | |
| 4-$SCF_2$H | 4-Cl | H | 4-$CO_2$Me | H | |
| 4-$CO_2$-i-$C_3H_7$ | 4-Cl | H | 4-$CO_2$Me | H | |
| 3,4-$OCF_2CF_2$[1] | 4-Cl | H | 4-$CO_2$Me | H | |
| 4-Cl | 4-F | H | 4-$CO_2$Me | H | m.p. 145 to 147°C |
| 4-$CF_2$H | 4-F | H | 4-$CO_2$Me | H | |
| 4-$OCF_2$H | 4-F | H | 4-$CO_2$Me | H | |
| 4-$SCF_2$H | 4-F | H | 4-$CO_2$Me | H | |
| 4-Br | 4-F | H | 4-$CO_2$Me | H | |
| 4-F | 4-F | H | 4-$CO_2$Me | H | |
| 3-$CF_3$-4-Cl | 4-F | H | 4-$CO_2$Me | H | |
| 4-$CF_3$ | 4-F | H | 4-$CO_2$Me | H | m.p. 148 to 150°C |
| 4-$OCF_3$ | 4-F | H | 4-$CO_2$Me | H | |
| 4-$CF_3$ | 4-Cl | H | 4-$CO_2$Et | H | m.p. 167 to 168°C |
| 4-Cl | 4-Cl | H | 4-$CO_2$Et | H | m.p. 144 to 147°C |

Table 1 (continued)

| $R_1$ | $R_2$ | $R_3$ | $R_5$ | Y | Phys. Prop. |
|---|---|---|---|---|---|
| 4-Br | 4-Cl | H | 4-$CO_2$Et | H | m.p. 158 to 160°C |
| 4-SMe | 4-Cl | H | 4-$CO_2$Et | H | m.p. 145 to 155°C |
| 4-$OCF_3$ | 4-Cl | H | 4-$CO_2$Et | H | |
| 4-$OCF_2H$ | 4-Cl | H | 4-$CO_2$Et | H | |
| 4-$CF_3$ | 4-Cl | H | 4-$CO_2H$ | H | |
| 4-Cl | 4-Cl | H | 4-$CO_2H$ | H | |
| 4-Br | 4-Cl | H | 4-$CO_2H$ | H | |
| 4-F | 4-Cl | H | 4-$CO_2H$ | H | |
| 4-$OCF_3$ | 4-Cl | H | 4-$CO_2H$ | H | |
| 4-$OCF_2H$ | 4-Cl | H | 4-$CO_2H$ | H | |
| 4-$CF_3$ | 4-Cl | H | 4-$CO_2$-i-Pr | H | m.p. 191 to 193°C |
| 4-Cl | 4-Cl | H | 4-$CO_2$-i-Pr | H | m.p. 191 to 193°C |
| 4-Br | 4-Cl | H | 4-$CO_2$-i-Pr | H | m.p. 204 to 206°C |
| 4-SMe | 4-Cl | H | 4-$CO_2$-i-Pr | H | m.p. 171 to 174°C |
| 4-$CF_3$ | 4-Cl | H | 4-$CO_2CH_2CF_3$ | H | m.p. 152 to 153.5°C |
| 4-Cl | 4-Cl | H | 4-$CO_2CH_2CF_3$ | H | m.p. 124 to 126°C |
| 4-Br | 4-Cl | H | 4-$CO_2CH_2CF_3$ | H | m.p. 133 to 134°C |
| 4-SMe | 4-Cl | H | 4-$CO_2CH_2CF_3$ | H | |
| 4-$CF_3$ | 3-Cl | H | 4-$CO_2$Me | H | m.p. 136 to 139°C |
| 4-Cl | 3-Cl | H | 4-$CO_2$Me | H | m.p. 188 to 189°C |
| 4-Br | 3-Cl | H | 4-$CO_2$Me | H | |
| 4-$OCF_2H$ | 3-Cl | H | 4-$CO_2$Me | H | |
| 4-$OCF_3$ | 3-Cl | H | 4-$CO_2$Me | H | |
| 3,4-di-Cl | 3-Cl | H | 4-$CO_2$Me | H | m.p. 100 to 104°C |
| 4-$CF_3$ | 3-Cl | H | 4-$CO_2$Et | H | |
| 4-Cl | 3-Cl | H | 4-$CO_2$Et | H | |
| 4-Br | 3-Cl | H | 4-$CO_2$Et | H | |
| 4-$OCF_2H$ | 3-Cl | H | 4-$CO_2$Et | H | |
| 4-$CF_3$ | 4-$CO_2$Me | H | 4-$CO_2$Me | H | m.p. 190 to 200°C |
| 4-Cl | 4-$CO_2$Me | H | 4-$CO_2$Me | H | m.p. 180 to 190°C(d) |

Table 1 (continued)

| $R_1$ | $R_2$ | $R_3$ | $R_5$ | Y | Phys. Prop. |
|---|---|---|---|---|---|
| 4-$CO_2$Et | 4-$CO_2$Me | H | 4-$CO_2$Me | H | m.p. 168 to 170°C |
| 3-$CF_3$ | 4-$CO_2$Me | H | 4-$CO_2$Me | H | m.p. 178 to 181°C |
| 4-$OCH_3$ | 4-$CO_2$Me | H | 4-$CO_2$Me | H | m.p. 151 to 153°C |
| 4-$CF_3$ | 4-OPh | H | 4-$CO_2$Me | H | m.p. 156 to 158°C |
| 4-Cl | 4-OPh | H | 4-$CO_2$Me | H | m.p. 175 to 177°C |
| 4-Br | 4-OPh | H | 4-$CO_2$Me | H | m.p. 172 to 176°C |
| 4-$CF_3$ | 3,4-di-Cl | H | 4-$CO_2$Me | H | m.p. 202 to 204°C |
| 4-Cl | 3,4-di-Cl | H | 4-$CO_2$Me | H | m.p. 187 to 189°C |
| 4-Br | 3,4-di-Cl | H | 4-$CO_2$Me | H | m.p. 191 to 192°C |
| 3,4-di-Cl | 3,4-di-Cl | H | 4-$CO_2$Me | H | m.p. 174.5 to 176°C |
| 4-$CF_3$ | 4-Cl | H | 4-$CO_2CH_2C\equiv CH$ | H | m.p. 108 to 112°C |
| 4-Cl | 4-Cl | H | 4-$CO_2CH_2C\equiv CH$ | H | m.p. 139 to 141°C |
| 4-Br | 4-Cl | H | 4-$CO_2CH_2C\equiv CH$ | H | m.p. 144 to 147°C |
| 4-$OCF_3$ | 4-Cl | H | 4-$CO_2CH_2C\equiv CH$ | H | |
| 4-$OCF_2H$ | 4-Cl | H | 4-$CO_2CH_2C\equiv CH$ | H | |
| 4-F | 4-Cl | H | 4-$CO_2CH_2C\equiv CH$ | H | |
| 4-$CF_3$ | 4-F | H | 4-$CO_2CH_2C\equiv CH$ | H | |
| 4-Cl | 4-F | H | 4-$CO_2CH_2C\equiv CH$ | H | |
| 4-Br | 4-F | H | 4-$CO_2CH_2C\equiv CH$ | H | |
| 4-$CF_3$ | 4-Br | H | 4-CO2$CH_2C\equiv CH$ | H | |
| 4-Cl | 4-Br | H | 4-$CO_2CH_2C\equiv CH$ | H | |
| 4-$CF_3$ | 4-$CF_3$ | H | 4-$CO_2$Me | H | m.p. 179 to 179.5°C |
| 4-Cl | 4-$CF_3$ | H | 4-$CO_2$Me | H | m.p. 155 to 158°C |
| 4-Br | 4-$CF_3$ | H | 4-$CO_2$Me | H | |
| 4-$OCF_2H$ | 4-$CF_3$ | H | 4-$CO_2$Me | H | |
| 4-$OCF_3$ | 4-$CF_3$ | H | 4-$CO_2$Me | H | |
| 4-$CF_3$ | 4-$OCF_2H$ | H | 4-$CO_2$Me | H | oil |
| 4-Cl | 4-$OCF_2H$ | H | 4-$CO_2$Me | H | |
| 4-Br | 4-$OCF_2H$ | H | 4-$CO_2$Me | H | |

Table 1 (continued)

| $R_1$ | $R_2$ | $R_3$ | $R_5$ | Y | Phys. Prop. |
|---|---|---|---|---|---|
| 4-$OCF_2H$ | 4-$OCF_2H$ | H | 4-$CO_2Me$ | H | |
| 4-$OCF_3$ | 4-$OCF_2H$ | H | 4-$CO_2Me$ | H | |
| 4-$CF_3$ | 4-$OCF_3$ | H | 4-$CO_2Me$ | H | m.p. 137 to 138.5°C |
| 4-Cl | 4-$OCF_3$ | H | 4-$CO_2Me$ | H | m.p. 105 to 107°C |
| 4-Br | 4-$OCF_3$ | H | 4-$CO_2Me$ | H | m.p. 106 to 108°C |
| 4-$OCF_2H$ | 4-$OCF_2H$ | H | 4-$CO_2Me$ | H | |
| 4-$OCF_3$ | 4-$OCF_2H$ | H | 4-$CO_2Me$ | H | |
| 4-$CF_3$ | 4-SMe | H | 4-$CO_2Me$ | H | m.p. 207.5 to 208.5°C |
| 4-Cl | 4-SMe | H | 4-$CO_2Me$ | H | m.p. 131 to 133°C |
| 4-Br | 4-SMe | H | 4-$CO_2Me$ | H | m.p. 160 to 163°C |
| 4-SMe | 4-SMe | H | 4-$CO_2Me$ | H | m.p. 177 to 179°C |
| 3-Cl-4-F | 4-SMe | H | 4-$CO_2Me$ | H | m.p. 175 to 178°C |
| 4-Cl | 4-$SO_2Me$ | H | 4-$CO_2Me$ | H | m.p. 138 to 142°C |
| 4-Br | 4-$SO_2Me$ | H | 4-$CO_2Me$ | H | m.p. 135 to 143°C |
| 4-$CF_3$ | 4-$SO_2Me$ | H | 4-$CO_2Me$ | H | m.p. 127 to 133°C |
| 4-Cl | 4-$NMe_2$ | H | 4-$CO_2Me$ | H | m.p. 136 to 150°C |
| 4-Br | 4-$NMe_2$ | H | 4-$CO_2Me$ | H | m.p. 152 to 160°C |
| 3-Cl-4-F | 4-$NMe_2$ | H | 4-$CO_2Me$ | H | m.p. 195 to 204°C |
| 4-$CF_3$ | 4-$NMe_2$ | H | 4-$CO_2Me$ | H | m.p. 190 to 200°C |
| 4-$CF_3$ | 3,4-di-F | H | 4-$CO_2Me$ | H | m.p. 189 to 190.5°C |
| 4-Cl | 3,4-di-F | H | 4-$CO_2Me$ | H | m.p. 170 to 171°C |
| 4-Br | 3,4-di-F | H | 4-$CO_2Me$ | H | m.p. 176.5 to 178°C |
| 4-$CO_2Et$ | 3,4-di-F | H | 4-$CO_2Me$ | H | m.p. 160 to 162°C |
| 4-Cl-3-$CF_3$ | 3,4-di-F | H | 4-$CO_2Me$ | H | m.p. 106 to 108°C(d) |
| 4-$CF_3$ | 4-Br | H | 4-$CO_2Me$ | H | m.p. 188 to 189.5°C |
| 4-Cl | 4-Br | H | 4-$CO_2Me$ | H | m.p. 152 to 154°C |
| 4-Br | 4-Br | H | 4-$CO_2Me$ | H | m.p. 154 to 155°C |
| 4-$OCF_2H$ | 4-Br | H | 4-$CO_2Me$ | H | |

Table 1 (continued)

| $R_1$ | R | $R_3$ | $R_5$ | Y | Phys. Prop. |
|---|---|---|---|---|---|
| 4-$OCF_3$ | 4-Br | H | 4-$CO_2Me$ | H | |
| 4-$OCH_3$ | 4-Br | H | 4-$CO_2Me$ | H | m.p. 178 to 180°C |
| 4-$CF_3$ | 4-Cl | H | 4-CONHMe | H | m.p. 222 to 224°C |
| 4-Cl | 4-Cl | H | 4-CONHMe | H | m.p. 189 to 191°C |
| 4-Br | 4-Cl | H | 4-CONHMe | H | |
| 4-F | 4-Cl | H | 4-CONHMe | H | m.p. 187 to 189°C |
| 3,4-di-Cl | 4-Cl | H | 4-CONHMe | H | m.p. 175 to 177°C |
| 4-$OCF_2H$ | 4-Cl | H | 4-CONHMe | H | |
| 4-$OCF_3$ | 4-Cl | H | 4-CONHMe | H | |
| 4-$CF_3$ | 4-Cl | H | 4-$CONMe_2$ | H | m.p. 124 to 125°C |
| 4-Cl | 4-Cl | H | 4-$CONMe_2$ | H | m.p. 175 to 177°C |
| 4-Br | 4-Cl | H | 4-$CONMe_2$ | H | m.p. 198 to 199.5°C |
| 4-$OCF_2H$ | 4-Cl | H | 4-$CONMe_2$ | H | |
| 4-$OCF_3$ | 4-Cl | H | 4-$CONMe_2$ | H | |
| 4-$CF_3$ | 4-Cl | H | 4-$CONEt_2$ | H | |
| 4-Cl | 4-Cl | H | 4-$CONEt_2$ | H | |
| 4-Br | 4-Cl | H | 4-$CONEt_2$ | H | |
| 4-$OCF_2H$ | 4-Cl | H | 4-$CONEt_2$ | H | |
| 4-$OCF_3$ | 4-Cl | H | 4-$CONEt_2$ | H | |
| 4-$CF_3$ | 4-Cl | H | 4-CONH-n-Bu | H | |
| 4-Cl | 4-Cl | H | 4-$CONH_2$ | H | |
| 4-Br | 4-Cl | H | 4-CONHEt | H | |
| 4-$OCF_2H$ | 4-Cl | H | 4-CONH-i-$C_3H_7$ | H | |
| 4-$OCF_3$ | 4-Cl | H | 4-$CONH_2$ | H | |
| 4-$CF_3$ | 4-Cl | H | 4-C(O)Me | H | m.p. 255 to 258°C |
| 4-Cl | 4-Cl | H | 4-C(O)Me | H | m.p. 264 to 266°C |
| 4-Br | 4-Cl | H | 4-C(O)Me | H | |
| 4-$OCF_2H$ | 4-Cl | H | 4-C(O)Me | H | |
| 4-$OCF_3$ | 4-Cl | H | 4-C(O)Me | H | |

Table 1 (continued)

| $R_1$ | $R_2$ | $R_3$ | $R_5$ | Y | Phys. Prop. |
|---|---|---|---|---|---|
| 4-$CF_3$ | 4-Cl | H | 4-C(O)Et | H | |
| 4-Cl | 4-Cl | H | 4-C(O)Et | H | |
| 4-Br | 4-Cl | H | 4-C(O)Et | H | |
| 4-$OCF_2H$ | 4-Cl | H | 4-C(O)Et | H | |
| 4-$OCF_3$ | 4-Cl | H | 4-C(O)Et | H | |
| 4-$CF_3$ | 4-Cl | H | 4-CHO | H | |
| 4-Cl | 4-Cl | H | 4-CHO | H | |
| 4-Br | 4-Cl | H | 4-CHO | H | |
| 4-$OCF_2H$ | 4-Cl | H | 4-CHO | H | |
| 4-$OCF_3$ | 4-Cl | H | 4-CHO | H | |
| 4-$CF_3$ | 4-Cl | H | 4-C(O)$CF_3$ | H | |
| 4-Cl | 4-Cl | H | 4-C(O)-n-Bu | H | |
| 4-Br | 4-Cl | H | 4-C(O)$(CH_2)_3$Cl | H | |
| 4-$OCF_2H$ | 4-Cl | H | 4-C(O)$CH_2$Br | H | |
| 4-$OCF_3$ | 4-Cl | H | 4-C(O)-i-$C_3H_7$ | H | |
| 4-$CF_3$ | 4-F | H | 4-C(O)Me | H | |
| 4-Cl | 4-F | H | 4-C(O)Me | H | |
| 4-Br | 4-F | H | 4-C(O)Me | H | |
| 4-$OCF_2H$ | 4-F | H | 4-C(O)Me | H | |
| 3,4-$OCF_2CF_2$[1] | 4-F | H | 4-C(O)Me | H | |
| 4-$OCF_3$ | 4-$OCF_2H$ | H | 4-C(O)Me | H | |
| 4-$CF_3$ | 4-$OCF_2H$ | H | 4-C(O)Me | H | |
| 4-Cl | 4-$OCF_2H$ | H | 4-C(O)Me | H | |
| 4-Br | 4-$OCF_2H$ | H | 4-C(O)Me | H | |
| 4-$OCF_2H$ | 4-$OCF_2H$ | H | 4-C(O)Me | H | |
| 3,4-$OCF_2CF_2$ | 4-$OCF_2H$ | H | 4-C(O)Me | H | |
| 4-$OCF_3$ | 4-$OCF_2H$ | H | 4-C(O)Me | H | |
| 4-$CF_3$ | 4-$CF_3$ | H | 4-C(O)Me | H | |

Table 1 (continued)

| $R_1$ | $R_2$ | $R_3$ | $R_5$ | Y | Phys. Prop. |
|---|---|---|---|---|---|
| 4-Cl | 4-$CF_3$ | H | 4-C(O)Me | H | |
| 4-Br | 4-$CF_3$ | H | 4-C(O)Me | H | |
| 4-$OCF_2H$ | 4-$CF_3$ | H | 4-C(O)Me | H | |
| 3,4-$OCF_2CF_2$[1] | 4-$CF_3$ | H | 4-C(O)Me | H | |
| 4-$OCF_3$ | 4-$CF_3$ | H | 4-C(O)Me | H | |
| 4-$CF_3$ | 4-F | H | 4-CHO | H | |
| 4-Cl | 4-F | H | 4-CHO | H | |
| 4-Br | 4-F | H | 4-CHO | H | |
| 4-$OCF_2H$ | 4-F | H | 4-CHO | H | |
| 4-$CF_3$ | 4-Cl | H | 4-$SO_2NMe_2$ | H | m.p. 199 to 202°C |
| 4-Cl | 4-Cl | H | 4-$SO_2NMe_2$ | H | m.p. 199 to 203°C |
| 4-Br | 4-Cl | H | 4-$SO_2NMe_2$ | H | |
| 4-$OCF_2H$ | 4-Cl | H | 4-$SO_2NMe_2$ | H | |
| 4-$OCF_3$ | 4-Cl | H | 4-$SO_2NMe_2$ | H | |
| 4-$CF_3$ | 4-Cl | H | 4-$SO_2NHMe$ | H | m.p. 209 to 212°C |
| 4-Cl | 4-Cl | H | 4-$SO_2NHMe$ | H | m.p. 231 to 234°C |
| 4-Br | 4-Cl | H | 4-$SO_2NHEt$ | H | |
| 4-$OCF_2H$ | 4-Cl | H | 4-$SO_2NH_2$ | H | |
| 4-$OCF_3$ | 4-Cl | H | 4-SO2$NEt_2$ | H | |
| 4-$CF_3$ | 4-F | H | 4-$SO_2NMe_2$ | H | |
| 4-Cl | 4-F | H | 4-$SO_2NMe_2$ | H | |
| 4-Br | 4-F | H | 4-$SO_2NMe_2$ | H | |
| 4-$OCF_2H$ | 4-F | H | 4-$SO_2NMe_2$ | H | |
| 4-$OCF_3$ | 4-F | H | 4-$SO_2NMe_2$ | H | |
| 4-$CF_3$ | 4-F | H | 4-$SO_2NH_2$ | H | |
| 4-Cl | 4-F | H | 4-$SO_2NHMe$ | H | |
| 4-Br | 4-F | H | 4-$SO_2NHEt$ | H | |
| 4-$OCF_2H$ | 4-F | H | 4-$SO_2NEt_2$ | H | |

Table 1 (continued)

| $R_1$ | $R_2$ | $R_3$ | $R_5$ | Y | Phys. Prop. |
|---|---|---|---|---|---|
| 4-$OCF_3$ | 4-F | H | 4-$SO_2NHMe$ | H | |
| 4-$CF_3$ | 4-F | H | 4-CONHMe | H | |
| 4-Cl | 4-F | H | 4-CONHMe | H | |
| 4-$CF_3$ | 4-F | H | 4-$CONMe_2$ | H | |
| 4-Cl | 4-F | H | 4-$CONMe_2$ | H | |
| 4-$CF_3$ | 4-$OCF_2H$ | H | 4-CONHMe | H | |
| 4-Cl | 4-$OCF_2H$ | H | 4-CONHMe | H | |
| 4-$CF_3$ | 4-$OCF_2H$ | H | 4-$CONMe_2$ | H | |
| 4-Cl | 4-$OCF_2H$ | H | 4-$CONMe_2$ | H | |
| 4-$CF_3$ | 4-$OCF_2H$ | H | 4-$CONH_2$ | H | |
| 4-Cl | 4-$OCF_2H$ | H | 4-$CONH_2$ | H | |
| 4-$CF_3$ | 4-F | H | 4-$SO_2NMe_2$ | H | |
| 4-Cl | 4-F | H | 4-$SO_2NMe_2$ | H | |
| 4-$CF_3$ | 4-F | H | 4-$SO_2NHMe$ | H | |
| 4-Cl | 4-F | H | 4-$SO_2NHMe$ | H | |
| 4-$CF_3$ | 4-$CF_3$ | H | 4-$SO_2NMe_2$ | H | m.p. 81 to 85°C |
| 4-Cl | 4-$CF_3$ | H | 4-$SO_2NMe_2$ | H | m.p. 85 to 88°C |
| 4-SMe | 4-$CF_3$ | H | 4-$SO_2NMe_2$ | H | m.p. 110 to 114°C |
| 4-$CF_3$ | 4-$CF_3$ | H | 4-$SO_2NHMe$ | H | |
| 4-Cl | 4-$CF_3$ | H | 4-$SO_2NHMe$ | H | |
| 4-$CF_3$ | 4-Cl | H | 4-$CS_2Me$ | H | |
| 4-Cl | 4-F | H | 4-$CS_2Me$ | H | |
| 4-Br | 4-$CF_3$ | H | 4-$CS_2Et$ | H | |
| 4-$OCF_2H$ | 4-$OCF_2H$ | H | 4-$CS_2Et$ | H | |
| 4-$OCF_3$ | 4-Cl | H | 4-$CSNMe_2$ | H | |
| 4-$CF_3$ | 4-F | H | 4-$CSNMe_2$ | H | |
| 4-Cl | 4-$CF_3$ | H | 4-CSNHMe | H | |
| 4-Br | 4-$OCF_2H$ | H | 4-CSNHMe | H | |

Table 1 (continued)

| $R_1$ | $R_2$ | $R_3$ | $R_5$ | Y | Phys. Prop. |
|---|---|---|---|---|---|
| 4-$OCF_2H$ | 4-Cl | H | 4-$CSNH_2$ | H | |
| 4-$CF_3$ | 4-F | H | 4-$CSNH_2$ | H | |
| 4-Cl | 4-$CF_3$ | H | 4-$CO_2$-allyl | H | |
| 4-Br | 4-$OCF_2H$ | H | 4-$CO_2$-allyl | H | |
| 4-$OCF_2H$ | 4-Cl | H | 4-$CO_2$-allyl | H | |
| 4-$OCF_3$ | 4-F | H | 4-$CO_2CH_2Ph$ | H | |
| 4-$CF_3$ | 4-$CF_3$ | H | 4-$CO_2CH_2Ph$ | H | |
| 4-Cl | 4-$OCF_2H$ | H | 4-$CO_2CH_2OMe$ | H | |
| 4-Br | 4-Cl | H | 4-$CO_2CH_2CN$ | H | |
| 4-$OCF_2H$ | 4-F | H | 4-$CO_2CH_2CH_2CN$ | H | |
| 4-Cl | 4-Cl | H | 4-$CO_2CH_2SMe$ | H | |
| 4-$CF_3$ | 4-F | H | 4-$CO_2CH_2CH_2NO_2$ | H | |
| 4-$CF_3$ | 4-Cl | 4-Cl | 3-$CO_2Me$ | H | m.p. 173 to 175°C |
| 4-$CF_3$ | 4-Cl | 4-F | 3-$CO_2Me$ | H | |
| 4-$CF_3$ | 4-Cl | 4-$OCF_2H$ | 3-$CO_2Me$ | H | |
| 4-$CF_3$ | 4-Cl | H | 3-$CO_2Me$ | H | m.p. 168 to 168.5°C |
| 4-Cl | 4-Cl | H | 3-$CO_2Me$ | H | m.p. 164 to 168°C |
| 4-Br | 4-Cl | H | 3-$CO_2Me$ | H | m.p. 160.5 to 163°C |
| 4-$CO_2Et$ | 4-Cl | H | 3-$CO_2Me$ | H | m.p. 186 to 187°C |
| 4-$CF_3$ | 4-Cl | 4-Cl | 2-$CO_2Me$ | H | |
| 4-$CF_3$ | 4-Cl | 4-F | 2-$CO_2Me$ | H | |
| 4-$CF_3$ | 4-Cl | 4-$OCF_2H$ | 2-$CO_2Me$ | H | |
| 4-$CF_3$ | 4-Cl | 4-Cl | 3-C(O)Me | H | |
| 4-$CF_3$ | 4-Cl | 4-F | 3-C(O)Me | H | |
| 4-$CF_3$ | 4-Cl | 4-$OCF_2H$ | 3-C(O)Me | H | |
| 4-$CF_3$ | 4-Cl | 4-Cl | 3-$CONME_2$ | H | |
| 4-$CF_3$ | 4-Cl | 4-F | 3-$CONME_2$ | H | |
| 4-$CF_3$ | 4-Cl | 4-$OCF_2H$ | 3-$CONME_2$ | H | |

Table 1 (continued)

| $R_1$ | $R_2$ | $R_3$ | $R_5$ | Y | Phys. Prop. |
|---|---|---|---|---|---|
| 4-$CF_3$ | 4-F | 4-Cl | 3-$CO_2$Me | H | |
| 4-$CF_3$ | 4-F | 4-F | 3-$CO_2$Me | H | |
| 4-$CF_3$ | 4-F | 4-$OCF_2$H | 3-$CO_2$Me | H | |
| 4-$CF_3$ | 4-$OCF_2$H | 4-Cl | 3-$CO_2$Me | H | |
| 4-$CF_3$ | 4-$OCF_2$H | 4-F | 3-$CO_2$Me | H | |
| 4-$CF_3$ | 4-$OCF_2$H | 4-$OCF_2$H | 3-$CO_2$Me | H | |
| 4-Cl | 4-Cl | 4-Cl | 3-$CO_2$Me | H | m.p. 187 to 188°C |
| 4-Br | 4-Cl | 4-Cl | 3-$CO_2$Me | H | |
| 4-Cl | 4-Cl | 4-Cl | 3-$CO_2$Me | H | |
| 4-Cl-3-$NO_2$ | 4-Cl | 4-Cl | 3-$CO_2$Me | H | |
| 4-$CF_3$ | 4-Cl | 3-Cl | 4-$CO_2$Me | H | |
| 4-$CF_3$ | 4-Cl | 3-F | 4-$CO_2$Me | H | |
| 4-$CF_3$ | 3,4-di-Cl | H | 4-CHO | H | |
| 4-$CF_3$ | 3,4-di-Cl | H | 4-$CONMe_2$ | H | |
| 4-$CF_3$ | 3,4-di-Cl | H | 4-$SO_2NMe_2$ | H | |
| 4-$CF_3$ | 3,4-di-Cl | H | 4-C(O)Me | H | |
| 4-$CF_3$ | 3-Cl-4-$OCF_2$H | H | 4-$CO_2$Me | H | |
| 4-$CF_3$ | 3-Cl-4-$OCF_2$H | H | 4-CHO | H | |
| 4-$CF_3$ | 3-Cl-4-$OCF_2$H | H | 4-$CONMe_2$ | H | |
| 4-$CF_3$ | 3-Cl-4-$OCF_2$H | H | 4-$SO_2NMe_2$ | H | |
| 4-$CF_3$ | 3-Cl-4-$OCF_2$H | H | 4-C(O)Me | H | |
| 4-$CF_3$ | 3-Cl-4-$OCF_3$ | H | 4-$CO_2$Me | H | |
| 4-$CF_3$ | 3-Cl-4-$OCF_3$ | H | 4-CHO | H | |
| 4-$CF_3$ | 3-Cl-4-$OCF_3$ | H | 4-$CONMe_2$ | H | |
| 4-$CF_3$ | 3-Cl-4-$OCF_3$ | H | 3-$SO_2NMe_2$ | H | |
| 4-$CF_3$ | 3-Cl-4-$OCF_3$ | H | 4-C(O)Me | H | |

Table 1 (continued)

| $R_1$ | $R_2$ | $R_3$ | $R_5$ | Y | Phys. Prop. |
|---|---|---|---|---|---|
| 4-$CF_3$ | 3,4-di-F | H | 4-$CONMe_2$ | H | |
| 4-$CF_3$ | 3,4-di-F | H | 4-CHO | H | |
| 4-$CF_3$ | 3,4-di-F | H | 4-$SO_2NMe_2$ | H | |
| 4-$CF_3$ | 3,4-di-F | H | 4-C(O)Me | H | |
| 4-$CF_3$ | 4-CN | H | 4-$CO_2Me$ | H | m.p. 169 to 170.5°C |
| 4-Cl | 4-CN | H | 4-$CO_2Me$ | H | m.p. 112 to 116°C |
| 4-Br | 4-CN | H | 4-$CO_2Me$ | H | |
| 4-$OCF_2H$ | 4-CN | H | 4-$CO_2Me$ | H | |
| 4-$OCF_3$ | 4-CN | H | 4-$CO_2Me$ | H | |
| 3,4-di-Cl | 4-CN | H | 4-$CO_2Me$ | H | m.p. 134 to 137°C |
| 4-$CF_3$ | 4-SCN | H | 4-$CO_2Me$ | H | |
| 4-$CF_3$ | 4-$NO_2$ | H | 4-$CO_2Me$ | H | |
| 4-$CF_3$ | 4-OEt | H | 4-$CO_2Me$ | H | |
| 4-$CF_3$ | 4-O-allyl | H | 4-$CO_2Me$ | H | |
| 4-$CF_3$ | 4-$OCH_2C(Cl)=CH_2$ | H | 4-$CO_2Me$ | H | m.p. 168 to 169°C |
| 4-$CF_3$ | 4-$SCF_2CF_2H$ | H | 4-$CO_2Me$ | H | |
| 4-$CF_3$ | 4-S(O)Me | H | 4-$CO_2Me$ | H | |
| 4-$CF_3$ | 4-$SO_2NMe_2$ | H | 4-$CO_2Me$ | H | |
| 4-$CF_3$ | 4-$NMe_2$ | H | 4-$CO_2Me$ | H | |
| 4-$CF_3$ | 4-$NEt_2$ | H | 4-$CO_2Me$ | H | |
| 4-Cl | 4-NHCHO | H | 4-$CO_2Me$ | H | |
| 4-Cl | 4-OC(O)NHMe | H | 4-$CO_2Me$ | H | |
| 4-Cl | 4-$NHSO_2Me$ | H | 4-$CO_2Me$ | H | |
| 4-Cl | 3,4-$OCH_2O$ | H | 4-$CO_2Me$ | H | |
| 4-Cl | 3,4-$OCF_2CF_2$[1] | H | 4-$CO_2Me$ | H | |
| 4-Cl | 3,4-$OCF_2CF_2O$[1] | H | 4-$CO_2Me$ | H | |
| 4-Cl | 4-OC(O)Me | H | 4-$CO_2Me$ | H | |
| 4-Cl | 4-$OSO_2Ph$ | H | 4-$CO_2Me$ | H | |

Table 1 (continued)

| $R_1$ | $R_2$ | $R_3$ | $R_5$ | Y | Phys. Prop. |
|---|---|---|---|---|---|
| 4-Cl | 4-Ph | H | 4-$CO_2$Me | H | |
| 4-Cl | 4-OPh | H | 4-$CO_2$Me | H | |
| 4-Cl | 4-allyl | H | 4-$CO_2$Me | H | |
| 4-Cl | 4-$CH_2$CN | H | 4-$CO_2$Me | H | |
| 4-$OCF_2CF_2$H | 4-Cl | H | 4-$CO_2$Me | H | |
| 4-$SCF_2CF_2$H | 4-Cl | H | 4-$CO_2$Me | H | |
| 4-CN | 4-Cl | H | 4-$CO_2$Me | H | |
| 3,4-di-Cl | 4-Cl | H | 4-$CO_2$Me | H | |
| 4-$CO_2$Me | 4-Cl | H | 4-$CO_2$Me | H | |
| 4-$CF_2$Cl | 4-Cl | H | 4-$CO_2$Me | H | |
| 4-$CO_2$Et | 4-Cl | H | 4-$CO_2$Me | H | m.p. 186 to 188°C |
| 4-$NMe_2$ | 4-Cl | H | 4-$CO_2$Me | H | |
| 4-$SO_2$Me | 4-Cl | H | 4-$CO_2$Me | H | |
| 4-SMe | 4-Cl | H | 4-$CO_2$Me | H | m.p. 191.5 to 193°C |
| 4-S(O)Me | 4-Cl | H | 4-$CO_2$Me | H | |
| 4-$SO_2NMe_2$ | 4-Cl | H | 4-$CO_2$Me | H | |
| 3,4-$CH_2CMe_2O$[1] | 4-Cl | H | 4-$CO_2$Me | H | |
| 3,4-$OCMe_2CH_2$[1] | 4-Cl | H | 4-$CO_2$Me | H | |
| 4-SCN | 4-Cl | H | 4-$CO_2$Me | H | |
| 4-$CF_3$-3-Cl | 4-Cl | H | 4-$CO_2$Me | H | |
| 4-Ph | 4-Cl | H | 4-$CO_2$Me | H | |
| 4-(4-Cl-Ph) | 4-Cl | H | 4-$CO_2$Me | H | |
| 4-OPh | 4-Cl | H | 4-CO2Me | H | |
| 4-$CH_2$Ph | 4-Cl | H | 4-$CO_2$Me | H | |
| 3-NO2-4-Cl | 4-Cl | H | 4-$CO_2$Me | H | m.p. 142 to 151°C(d) |
| 2-OMe-4-Cl | 4-Cl | H | 4-$CO_2$Me | H | m.p. 180 to 185°C |
| 3-Cl-4-F | 4-Cl | H | 4-$CO_2$Me | H | m.p. 165 to 168°C |
| 3-$CF_3$-4-Cl | 4-Cl | H | 4-$CO_2$Me | H | m.p. 120 to 129°C |
| 3,4-di-Cl | 4-Cl | H | 4-$CO_2$Me | H | m.p. 235 to 243°C |

Table 1 (continued)

| $R_1$ | $R_2$ | $R_3$ | $R_5$ | Y | Phys. Prop. |
|---|---|---|---|---|---|
| 4-$CF_3$ | 4-Cl | H | 4-$CO_2$Me | Me | m.p. 92 to 98°C |
| 4-Cl | 4-Cl | H | 4-$CO_2$Me | Me | m.p. 90 to 95°C |
| 4-Br | 4-Cl | H | 4-$CO_2$Me | Me | m. p. 82 to 86°C |
| 4-$OCF_2$H | 4-Cl | H | 4-$CO_2$Me | Me | |
| 4-$OCF_3$ | 4-Cl | H | 4-$CO_2$Me | Me | |
| 4-$CF_3$ | 4-F | H | 4-$CO_2$Me | Me | |
| 4-Cl | 4-F | H | 4-$CO_2$Me | Me | |
| 4-$OCF_2$H | 4-F | H | 4-$CO_2$Me | Me | |
| 4-$OCF_3$ | 4-F | H | 4-$CO_2$Me | Me | |
| 4-$CF_3$ | 4-Cl | H | 4-$CO_2$Me | C(O)Me | |
| 4-Cl | 4-Cl | H | 4-$CO_2$Me | C(O)Me | |
| 4-$OCF_2$H | 4-Cl | H | 4-$CO_2$Me | C(O)Me | |
| 4-$OCF_3$ | 4-Cl | H | 4-$CO_2$Me | C(O)Me | |
| 4-$CF_3$ | 4-F | H | 4-$CO_2$Me | C(O)Me | |
| 4-Cl | 4-F | H | 4-$CO_2$Me | C(O)Me | |
| 4-$OCF_2$H | 4-F | H | 4-$CO_2$Me | C(O)Me | |
| 4-$OCF_3$ | 4-F | H | 4-$CO_2$Me | C(O)Me | |
| 4-$CF_3$ | 4-Cl | H | 4-$CO_2$Me | $CO_2$Me | |
| 4-Cl | 4-Cl | H | 4-$CO_2$Me | $CO_2$Me | |
| 4-$OCF_2$H | 4-Cl | H | 4-$CO_2$Me | $CO_2$Me | |
| 4-$OCF_3$ | 4-Cl | H | 4-$CO_2$Me | $CO_2$Me | |
| 4-$CF_3$ | 4-F | H | 4-$CO_2$Me | $CO_2$Me | |
| 4-Cl | 4-F | H | 4-$CO_2$Me | $CO_2$Me | |
| 4-$OCF_2$H | 4-F | H | 4-$CO_2$Me | $CO_2$Me | |
| 4-$CF_3$ | 4-Cl | H | 4-$CO_2$Me | CHO | |
| 4-Cl | 4-Cl | H | 4-$CO_2$Me | CHO | |
| 4-$OCF_2$H | 4-Cl | H | 4-$CO_2$Me | CHO | |
| 4-$OCF_3$ | 4-Cl | H | 4-$CO_2$Me | CHO | |

Table 1 (continued)

| $R_1$ | $R_2$ | $R_3$ | $R_5$ | Y | Phys. Prop. |
|---|---|---|---|---|---|
| 4-$CF_3$ | 4-Cl | H | 4-$CO_2$Me | $CO_2$Et | |
| 4-Cl | 4-Cl | H | 4-$CO_2$Me | $CO_2$Et | |
| 4-$OCF_2H$ | 4-Cl | H | 4-$CO_2$Me | $CO_2$Et | |
| 4-$OCF_3$ | 4-Cl | H | 4-$CO_2$Me | $CO_2$Et | |
| 4-$CF_3$ | 4-Cl | H | 4-$CO_2$Me | C(O)Et | |
| 4-Cl | 4-Cl | H | 4-$CO_2$Me | C(O)Et | |
| 4-$OCF_2H$ | 4-Cl | H | 4-$CO_2$Me | C(O)Et | |
| 4-$OCF_3$ | 4-Cl | H | 4-$CO_2$Me | C(O)Et | |
| 4-$CF_3$ | 4-Cl | H | 4-$CO_2$Me | C(O)$CH_2$Cl | |
| 4-Cl | 4-Cl | H | 4-$CO_2$Me | C(O)$CH_2$Cl | |
| 4-$OCF_2H$ | 4-Cl | H | 4-$CO_2$Me | C(O)$CH_2$Cl | |
| 4-$OCF_3$ | 4-Cl | H | 4-$CO_2$Me | C(O)$CH_2$Cl | |
| 4-$CF_3$ | 4-Cl | H | 4-$CO_2$Me | SMe | |
| 4-Cl | 4-Cl | H | 4-$CO_2$Me | SMe | |
| 4-$CF_3$ | 4-Cl | H | 4-$CO_2$Me | SPh | |
| 4-Cl | 4-Cl | H | 4-$CO_2$Me | SPh | |
| 4-$CF_3$ | 4-Cl | H | 4-$CO_2$Me | n-Bu | |
| 4-$CF_3$ | 4-F | H | 4-$CO_2$Et | Me | |
| 4-$CF_3$ | 4-F | H | 4-$CONMe_2$ | Me | |
| 4-$CF_3$ | 4-F | H | 4-CHO | Me | |
| 4-$CF_3$ | 4-F | H | 4-C(O)Me | Me | |
| 4-$CF_3$ | 4-F | H | 4-$SO_2$NMe | CHO | |
| 4-$CF_3$ | 4-F | H | 4-$CO_2$Et | CHO | |
| 4-$CF_3$ | 4-F | H | 4-CHO | CHO | |
| 4-$CF_3$ | 4-F | H | 4-C(O)Me | $CO_2$Me | |
| 4-$CF_3$ | 4-F | H | 4-$SO_2NMe_2$ | $CO_2$Me | |
| 4-$CF_3$ | 4-F | H | 4-$CO_2$Et | $CO_2$Me | |
| 4-$CF_3$ | 4-F | H | 4-$CONMe_2$ | $CO_2$Me | |

Table 1 (continued)

| $R_1$ | $R_2$ | $R_3$ | $R_5$ | Y | Phys. Prop. |
|---|---|---|---|---|---|
| 4-$CF_3$ | 4-F | H | 4-CHO | SMe | |
| 4-$CF_3$ | 4-F | H | 4-C(O)Me | SMe | |
| 4-$CF_3$ | 4-F | H | 4-$SO_2NMe_2$ | SMe | |
| 4-$CF_3$ | H | H | 4-$CO_2Et$ | SMe | |

1. $R_1$ or $R_2$ substitutents designated as disubstituted with two open termini are bound to phenyl to form a bicyclic ring such that the first numerical designation defines the position of the first listed terminus and the second numerical designation defines the position of the other terminus.

TABLE 2

| $R_1$ | $R_2$ | $R_3$ | $R_5$ | Y | Phys. Prop. |
|---|---|---|---|---|---|
| 4-$CF_3$ | 4-Cl | H | 4-$CO_2Me$ | H | semi-solid |
| 4-Cl | 4-Cl | H | 4-$CO_2Me$ | H | |
| 4-Br | 4-Cl | H | 4-$CO_2Me$ | H | |
| 4-$OCF_2H$ | 4-F | H | 4-$CO_2Me$ | H | |
| 4-$OCF_3$ | 4-F | H | 4-$CO_2Me$ | H | |
| 4-F | 4-F | H | 4-$CO_2Me$ | H | |
| 3,4-$OCF_2CF_2$[1] | 4-$OCF_2H$ | H | 4-$SO_2NMe_2$ | H | |
| 4-$CO_2$-i-$C_3H_7$ | 4-$OCF_2H$ | H | 4-$SO_2NMe_2$ | H | |
| 4-$CF_3$ | 4-$OCF_2H$ | H | 4-$SO_2NMe_2$ | H | |
| 4-Cl | 4-$OCF_3$ | H | 4-$SO_2NMe_2$ | H | |
| 4-Br | 4-$OCF_3$ | H | 4-$SO_2NMe_2$ | H | |
| 4-$OCF_2H$ | 4-$OCF_3$ | H | 4-$SO_2NMe_2$ | H | |
| 4-$OCF_3$ | 4-Br | H | 4-C(O)Me | H | |
| 4-F | 4-Br | H | 4-C(O)Me | H | |
| 3,4-$OCF_2CF_2$[1] | 4-Br | H | 4-C(O)Me | H | |
| 4-$CO_2$-i-$C_3H_7$ | 4-$CF_3$ | H | 4-C(O)Me | H | |
| 4-$CF_3$ | 4-$CF_3$ | H | 4-C(O)Me | H | |
| 4-Cl | 4-$CF_3$ | H | 4-C(O)Me | H | |
| 4-Br | 4-$NMe_2$ | H | 4-$CO_2Me$ | H | |
| 4-$OCF_2H$ | 4-$NO_2$ | H | 4-$CO_2Me$ | H | |
| 4-$OCF_3$ | 4-OMe | H | 4-$CO_2Me$ | H | |
| 4-$CF_3$ | 4-Br | H | 4-$CO_2Me$ | H | m.p. 188 to 189.5 °C |
| 4-$CF_3$ | 4-$CO_2Me$ | H | 4-$CO_2Me$ | H | m.p. 173 to 175 °C |
| 4-F | 4-Cl | H | 4-$CO_2Me$ | H | |
| 4-$CF_3$ | 4-Cl | H | 4-$CONMe_2$ | H | |
| 4-Cl | 4-Cl | H | 4-$CONMe_2$ | H | |
| 4-Br | 4-Cl | H | 4-$CONMe_2$ | H | |
| 4-$OCF_2H$ | 4-F | H | 4-$CONMe_2$ | H | |
| 4-$OCF_3$ | 4-F | H | 4-$CONMe_2$ | H | |

TABLE 2 (continued)

| $R_1$ | $R_2$ | $R_3$ | $R_5$ | Y | Phys. Prop. |
|---|---|---|---|---|---|
| 4-F | 4-F | H | 4-$CONME_2$ | H | |
| 3,4-$OCF_2CF_2$[1] | 4-$OCF_2H$ | H | 4-CHO | H | |
| 4-$CO_2$-i-$C_3H_7$ | 3-$OCF_2H$ | H | 4-CHO | H | |
| 4-$CF_3$ | 4-$OCF_2H$ | H | 4-CHO | H | |
| 4-Cl | 4-$OCF_3$ | H | 4-CHO | H | |
| 4-Br | 4-$OCF_3$ | H | 4-CHO | H | |
| 4-$OCF_2H$ | 4-$OCF_3$ | H | 4-$CO_2H$ | H | |
| 4-$OCF_3$ | 4-Br | H | 4-$CO_2H$ | H | |
| 4-F | 4-Br | H | 4-$CO_2H$ | H | |
| 3,4-$OCF_2CF_2$[1] | 4-Br | H | 4-$CO_2H$ | H | |
| 4-$CO_2$-i-$C_3H_7$ | 4-$CF_3$ | H | 4-$CO_2H$ | H | |
| 4-Cl | 4-$CF_3$ | H | 4-$CO_2Et$ | H | m.p. 170 to 173 °C |
| 4-Cl | 4-$CF_3$ | H | 4-$CO_2Et$ | H | |
| 4-Br | 4-$NMe_2$ | H | 4-$CO_2Et$ | H | |
| 4-$OCF_2H$ | 3-$NO_2$ | H | 4-$CO_2Et$ | H | |
| 4-$OCF_3$ | 4-OMe | H | 4-$CO_2Et$ | H | |
| 4-F | 4-Cl | H | 4-$CO_2Et$ | H | |

TABLE 3

| $R_1$ | $R_2$ | $R_3$ | $R_5$ | $R_6$ | $R_7$ | Phys. Prop. |
|---|---|---|---|---|---|---|
| 4-$CF_3$ | 4-Cl | H | 4-$CO_2Me$ | Me | H | m.p. 92 to 98 |
| 4-Cl | 4-Cl | H | 4-$CO_2Me$ | Me | H | m.p. 90 to 95 |
| 4-Br | 4-Cl | H | 4-$CO_2Me$ | Me | H | m.p. 82 to 86 |
| 4-$OCF_2H$ | 4-Cl | H | 4-$CO_2Me$ | Me | H | |
| 4-$OCF_3$ | 4-Cl | H | 4-$CO_2Me$ | Me | H | |
| 4-$CF_3$ | 4-Cl | H | 4-C(O)Me | Me | H | |
| 4-Cl | 4-Cl | H | 4-C(O)Me | Me | H | |
| 4-Br | 4-Cl | H | 4-C(O)Me | Me | H | |
| 4-$OCF_2H$ | 4-Cl | H | 4-C(O)Me | Me | H | |
| 4-$OCF_3$ | 4-Cl | H | 4-C(O)Me | Me | H | |
| 4-$CF_3$ | 4-F | H | 4-$CO_2Me$ | Me | H | |
| 4-Cl | 4-F | H | 4-$CO_2Me$ | Me | H | |
| 4-Br | 4-F | H | 4-$CO_2Me$ | Me | H | |
| 4-$OCF_2H$ | 4-F | H | 4-$CO_2Me$ | Me | H | |
| 4-$OCF_3$ | 4-F | H | 4-$CO_2Me$ | Me | H | |
| 4-$CF_3$ | 4-F | H | 4-$CONMe_2$ | Me | H | |
| 4-Br | 4-F | H | 4-$CONMe_2$ | Me | H | |
| 4-$OCF_2H$ | 4-F | H | 4-$CONMe_2$ | Me | H | |
| 4-$OCF_3$ | 4-F | H | 4-$CONMe_2$ | Me | H | |
| 4-$CF_3$ | 4-Cl | H | 4-$CO_2Me$ | Me | Me | |
| 4-Cl | 4-Cl | H | 4-$CO_2Me$ | Me | Me | |
| 4-Br | 4-Cl | H | 4-$CO_2Me$ | Me | Me | |
| 4-$OCF_2H$ | 4-Cl | H | 4-$CO_2Me$ | Me | Me | |
| 4-$OCF_3$ | 4-Cl | H | 4-$CO_2Me$ | Me | Me | |
| 4-$CF_3$ | 4-Cl | H | 4-$SO_2NMe_2$ | Me | Me | |
| 4-Cl | 4-Cl | H | 4-$SO_2NMe_2$ | Me | Me | |
| 4-Br | 4-Cl | H | 4-$SO_2NMe_2$ | Me | Me | |
| 4-$OCF_2H$ | 4-Cl | H | 4-$SO_2NMe_2$ | Me | Me | |

TABLE 3 (continued)

| $R_1$ | $R_2$ | $R_3$ | $R_5$ | $R_6$ | $R_7$ | Phys. Prop. |
|---|---|---|---|---|---|---|
| 4-$OCF_3$ | 4-Cl | H | 4-$SO_2NMe_2$ | Me | Me | |
| 4-$CF_3$ | 4-F | H | 4-$CO_2Me$ | Me | Me | |
| 4-Cl | 4-F | H | 4-$CO_2Me$ | Me | Me | |
| 4-Br | 4-F | H | 4-$CO_2Me$ | Me | Me | |
| 4-$OCF_2H$ | 4-F | H | 4-$CO_2Me$ | Me | Me | |
| 4-$OCF_3$ | 4-F | H | 4-$CO_2Me$ | Me | Me | |
| 4-$CF_3$ | 4-F | H | 4-$CO_2H$ | Me | Me | |
| 4-Cl | 4-F | H | 4-$CO_2H$ | Me | Me | |
| 4-Br | 4-F | H | 4-$CO_2H$ | Me | Me | |
| 4-$OCF_2H$ | 4-F | H | 4-$CO_2H$ | Me | Me | |
| 4-$OCF_3$ | 4-F | H | 4-$CO_2H$ | Me | Me | |
| 4-$CF_3$ | 4-Cl | H | 4-$CO_2Me$ | Et | H | |
| 4-$CF_3$ | 4-F | H | 4-$CO_2Me$ | Et | H | |
| 4-$CF_3$ | 4-$OCF_2H$ | H | 4-$CO_2Me$ | Et | H | |
| 4-$CF_3$ | 4-Cl | H | 4-$CO_2Et$ | Et | H | |
| 4-$CF_3$ | 4-F | H | 4-$CO_2Et$ | Et | H | |
| 4-$CF_3$ | 4-$OCF_2H$ | H | 4-$CO_2Et$ | Et | H | |
| 4-$CF_3$ | 4-Cl | H | 4-$CO_2Me$ | i-Pr | H | |
| 4-$CF_3$ | 4-F | H | 4-$CO_2Me$ | i-Pr | H | |
| 4-$CF_3$ | 4-$OCF_2H$ | H | 4-$CO_2Me$ | i-Pr | H | |
| 4-$CF_3$ | 4-Cl | H | 4-$CO_2Et$ | i-Pr | H | |
| 4-$CF_3$ | 4-F | H | 4-$CO_2Et$ | n-Pr | H | |
| 4-$CF_3$ | 4-$OCF_2H$ | H | 4-$CO_2Et$ | n-Pr | H | |
| 4-$CF_3$ | 4-Cl | H | 4-$CO_2Me$ | n-Pr | H | |
| 4-$CF_3$ | 4-F | H | 4-$CO_2Me$ | n-Pr | H | |
| 4-$CF_3$ | 4-$OCF_2H$ | H | 4-$CO_2Me$ | n-Pr | H | |

TABLE 3 (continued)

| R | $R_2$ | $R_3$ | $R_5$ | $R_6$ | $R_7$ | Phys. Prop. |
|---|---|---|---|---|---|---|
| 4-$CF_3$ | 4-Cl | H | 4-$CO_2$Et | n-Bu | H | |
| 4-$CF_3$ | 4-F | H | 4-$CO_2$Et | n-Bu | H | |
| 4-$CF_3$ | 4-$OCF_2$H | H | 4-$CO_2$Et | n-Bu | H | |
| 4-$CF_3$ | 4-Cl | H | 4-$CO_2$Me | n-Bu | H | |
| 4-$CF_3$ | 4-F | H | 4-$CO_2$Me | n-Bu | H | |

Formulation and Use

The compounds of this invention will generally be used in formulation with a carrier comprising a liquid or solid diluent or an organic solvent. Useful formulations of the compounds of Formula I can be prepared in conventional ways. They include dusts, granules, pellets, solutions, suspensions, emulsions, wettable powders, emulsifiable concentrates, dry flowables and the like. Many of these can be applied directly. Sprayable formulations can be extended in suitable media and used at spray volumes of from about one to several hundred liters per hectare. High strength compositions are primarily used as intermediates for further formulation. The formulations, broadly, contain about 1% to 99% by weight of active ingredient(s) and at least one of a) about 0.1% to 20% surfactant(s) and b) about 5% to 99% solid or liquid diluent(s). More specifically, they will contain these ingredients in the following approximate proportions:

| | Percent by Weight | | |
|---|---|---|---|
| | Active Ingredient | Diluent(s) | Surfactant(s) |
| Wettable Powders | 25-90 | 0-74 | 1-10 |
| Oil Suspensions, Emulsions, Solutions, (including Emulsifiable Concentrates) | 5-50 | 40-95 | 0-15 |
| Dusts | 1-25 | 70-99 | 0-5 |
| Granules and Pellets | 1-95 | 5-99 | 0-15 |
| High Strength Compositions | 90-99 | 0-10 | 0-2 |

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0° C. "McCutcheon's Detergents and Emulsifiers Annual:, MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking corrosion, microbiological growth, etc. Preferably, ingredients should be approved by the U.S. Environmental Protection Agency for the use intended.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pp. 147 and following, and "Perry's Chemical Engineer's Handbook", 4th Ed., McGraw-Hill, New York, 1963, pages 8 to 59 and following.

Examples of useful formulations of compounds of the present invention are as follows:

## Example 2

### Emulsifiable Concentrate

N,3-bis[4-(trifluoromethyl)phenyl]-4-[4-(dimethylamino)-sulfonyl]phenyl]-4,5-dihydro-1H-pyrazole-1-carboxamide 10%
blend of oil soluble sulfonates and polyoxyethylene ethers 4%
isophorone 86%

The ingredients are combined and stirred with gentle warming to speed solution. A fine screen filter is included in packaging operation to insure the absence of any extraneous undissolved material in the product.

## Example 3

### Wettable Powder

methyl 4-[3-(4-chlorophenyl)-4,5-dihydro-1-[[4-(trifluoromethyl)phenylamino]carbonyl]-1H-pyrazol-4-yl]-benzoate 80%
sodium alkylnaphthalenesulfonate 2%
sodium ligninsulfonate 2%
synthetic amorphous silica 3%
kaolinite 3%

The active ingredient is blended with the inert materials in a blender. After grinding in a hammermill, the material is reblended and sifted through a U.S.S. 50 mesh screen and packaged.

Example 4

Dust

wettable powder of Example 2 5%
pyrophyllite (powder) 95%

The wettable powder and the pyrophyllite diluent are thoroughly blended and then packaged. The product is suitable for use as a dust.

Example 5

Granule

methyl 4-[3-(4-chlorophenyl)-4,5-dihydro-1-[[4-(trifluoromethyl)phenylamino]carbonyl]-1H-pyrazol-4-yl]-benzoate 10%
attapulgite granules (low volative matter, 0.71/0.30 mm; U.S.S. No. 25-50 sieves) 90%

The active ingredient is dissolved in a suitable solvent and sprayed onto dedusted attapulgite granules in a double cone blender. The granules are warmed to drive off solvent, cooled and packaged.

Example 6

Granule

wettable powder of Example 2 15%
gypsum 69%
potassium sulfate 16%

The ingredients are blended in a rotating mixer and water is sprayed on to accomplish granulation. When most of the material has reached the desired range of 0.1 to 0.42 mm (U.S.S. No. 18 to 40 sieves), the granules are removed, dried, and screened. Oversize material is crushed to produce additional material in the desired range. These granules contain 12% active ingredient.

Example 7

Solution

methyl 4-[3-(4-chlorophenyl)-4,5-dihydro-1-[[4-(trifluoromethyl)phenylamino]carbonyl]-1H-pyrazol-4-yl]-benzoate 15%
4-butyrolactone 85%

The ingredients are combined and stirred to produce a solution suitable for direct, low volume application.

Example 8

Oil Suspension

methyl 4-[3-(4-chlorophenyl)-4,5-dihydro-1-[[4-(trifluoromethyl)phenylamino]carbonyl]-1H-pyrazol-4-yl]-benzoate 25%
polyoxyethylene sorbitol hexaoleate 5%
highly aliphatic hydrocarbon oil 70%

The ingredients are ground together in a sand mill until the solid particles have been reduced to under 5 microns. The resulting thick suspension may be applied directly, but preferable after being extended with oils or emulsified in water.

Example 9

Wettable Powder

methyl 4-[3-(4-chlorophenyl)-4,5-dihydro-1-[[4-(trifluoromethyl)phenylamino]carbonyl]-1H-pyrazol-4-yl]-benzoate 20%
sodium alkylnaphthalenesulfonate 4%
sodium lignosulfonate 4%
low viscosity methyl cellulose 3%
attapulgite 69%

The active ingredient is blended with the inert materials in a blender. After griding in a hammermill to produce particles essentially all below 100 microns, the material is reblended and sifted through a U.S.S. 50 mesh screen and packaged.

Compounds of Formula I can also be mixed with one or more other insecticides, fungicides, nematocides, bactericides, acaricides, or other biologically active compounds to form a multicomponent pesticide giving an even broader spectrum of effective agricultural protection. Examples of other agricultural protectants with which compounds of the present invention can be mixed or formulated are:

Insecticides:

3-hydroxy-N-methylcrotonamide(dimethylphosphate)ester (monocrotophos)
methylcarbamic acid, ester with 2,3-dihydro-2,2-dimethyl-7-benzofuranol (carbofuran)
O-[2,4,5-trichloro-a-(chloromethyl)benzyl]phosphoric acid, O′,O′-dimethyl ester (tetrachlorvinphos)
2-mercaptosuccinic acid, diethyl ester, S-ester with thionophosphoric acid, dimethyl ester (malathion)
phosphorothioic acid, O,O-dimethyl, O-p-nitrophenyl ester (methyl parathion)
methylcarbamic acid, ester with a-naphthol (carbaryl)

methyl O-(methylcarbamoyl)thiolacetohydroxamate (methomyl)
N′-(4-chloro-o-tolyl)-N,N-dimethylformamidine (chlordimeform)
O,O-diethyl-O-(2-isopropyl-4-methyl-6-pyrimidylphos-phorothioate (diazinon)
octachlorocamphene (toxaphene)
O-ethyl O-p-nitrophenyl phenylphosphonothioate (EPN)
(S)-a-cyano-m-phenoxybenzyl(1R,3R)-3-(2,2-dibromovinyl)-2,2-dimethylcyclopropanecarboxylate (deltamethrin)
Methyl N′,N′-dimethyl-N-[(methylcarbamoyl)oxy]-1-thio-oxamimidate (oxamyl)
cyano(3-phenoxyphenyl)-methyl-4-chloro-a-(1-methylethyl)benzeneacetate (fenvalerate)
(3-phenoxyphenyl)methyl(±)-cis,trans-3-(2,2-dichloroethenyl)-2,2-dimethylcyclopropanecarboxylate (permethrin)
a-cyano-3-phenoxybenzyl 3-(2,2-dichlorovinyl)-2,2-dimethylcyclopropane carboxylate (cypermethrin)
O-ethyl-S-(p-chlorophenyl)ethylphosphonodithioate (profenofos)
phosphorothiolothionic acid,
O-ethyl-O-[4-(methylthio)-phenyl]-S-n-propyl ester (sulprofos).

Additional insecticides are listed hereafter by their common names: triflumuron, diflubenzuron, methoprene, buprofezin, thiodicarb, acephate, azinphos-methyl, chlorpyrifos, dimethoate, fonophos, isofenphos, methidathion, methamidiphos, monocrotophos, phosmet, phosphamidon, phosalone, pirimicarb, phorate, profenofos, terbufos, trichlorfon, methoxychlor, bifenthrin, biphenate, cyfluthrin, fenpropathrin, fluvalinate, flucythrinate, tralomethrin, metaldehyde and rotenone.

Fungicides:

methyl 2-benzimidazolecarbamate (carbendazim)
tetramethylthiuram disulfide (thiuram)
n-dodecylguanidine acetate (dodine)
manganese ethylenebisdithiocarbamate (maneb)
1,4-dichloro-2,5-dimethoxybenzene (chloroneb)
methyl 1-(butylcarbamoyl)-2-benzimidazolecarbamate (benomyl)
1-[2-(2,4-dichlorophenyl)-4-propyl-1,3-dioxolan-2-ylmethyl]-1H-1,2,4-triazole (propiconazole)
2-cyano-N-ethylcarbamoyl-2-methoxyiminoacetamide (cymoxanil)
1-(4-chlorophenoxy)-3,3-dimethyl-1-(1H-1,2,4-triazol-1-yl)-2-butanone (triadimefon)
N-(trichloromethylthio)tetrahydrophthalimide (captan)
N-(trichloromethylthio)phthalimide (folpet)
1-[[[bis(4-fluorophenyl)][methyl]silyl]methyl]-1H-1,2,4-triazole.

Nematocides:

S-methyl 1-(dimethylcarbamoyl)-N-(methylcarbamoyloxy)-thioformimidate
S-methyl 1-carbamoyl-N-(methylcarbamoyloxy)thioformimidate
N-isopropylphosphoramidic acid, O-ethyl O′-[4-(methylthio)-m-tolyl]diester (fenamiphos).

Bactericides:

tribasic copper sulfate
streptomycin sulfate.

Acaricides:

senecioic acid, ester with 2-sec-butyl-4,6-dinitrophenol (binapacryl)
6-methyl-1,3-dithiolo[4,5-β]quinoxalin-2-one (oxythioquinox)
ethyl 4,4′-dichlorobenzilate (chlorobenzilate)
1,1-bis(p-chlorophenyl)-2,2,2-trichloroethanol (dicofol)
bis(pentachloro-2,4-cyclopentadien-1-yl) (dienochlor)
tricyclohexyltin hydroxide (cyhexatin)
trans-5-(4-chlorophenyl)-N-cyclohexyl-4-methyl-2-oxothiazolidine-3-carboxamide (hexythiazox)
amitraz
propargite
fenbutatin-oxide
bisclofentezin.

Biological

Bacillus thuringiensis
Avermectin B.

Utility

The compounds of the present invention exhibit activity against a wide spectrum of foliar and soil inhabiting insects. Those skilled in the art will recognize that not all compounds will be equally effective against all insects, but compounds of this invention display control of many of the economically important pest species of the insect orders Lepidoptera, Homoptera, and Coleoptera among many others. The specific species for which control is exemplified below are: fall armyworm, Spodoptera frugiperda; boll weevil, Anthonomus grandis; European corn borer, Ostrinia nubilalis; southern corn rootworm, Diabrotica undecimpunctata howardi; aster leafhopper, Macrosteles fascifrons. The pest control afforded by the compounds of the present invention is not limited, however, to these species.

Application

Insects are controlled and agricultural crops are protected by applying one or more of the Formula I compounds of this invention, in an effective amount, to the locus of infestation, to the area to be protected (the environment of the pests), or directly on the pests to be controlled. A preferred method of application is by spraying with spray equipment that distributes the compound on the foliage, in the soil, or to the plant part that is infested or needs to be protected. Alternatively, granular formulations of these compounds can be applied to soil or foliage or, optionally, incorporated into the soil. Either aerial or ground application can be used.

The pyrazoline compound(s) of this invention can be applied in its (their) pure state, but most often application will be of a formulation comprising one or more compounds of this invention, in an agriculturally suitable carrier or diluent. A most preferred method of application involves spraying a water dispersion or refined oil solution of the compounds.

The rate of application of the Formula I compounds required for effective control will depend on such factors as the insect species population size, the pest's life stage, insect size, its location, the host crop, the host stage, time of year of application, placement of the insecticide, temperature conditions, and others. In general, application rates of 0.05 to 2 kg of active ingredient per hectare are sufficient to provide effective control in large scale field operations under normal circumstances, but as little as 0.01 kg/hectare may be sufficient or as much as 8 kg/hectare may be required, depending upon the factors listed above.

Examples 10 to 15 demonstrate the control efficacy of compounds of Formula I on specific insect pests. Compounds 1 through 115 in these tests are described in Table 4. In each of Compounds 1 to 115, $R_6$, $R_7$ and Y are hydrogen and X is oxygen with the following exceptions: in compound 81, Y is methyl and in compounds 104 to 106, $R_6$ is methyl.

TABLE 4

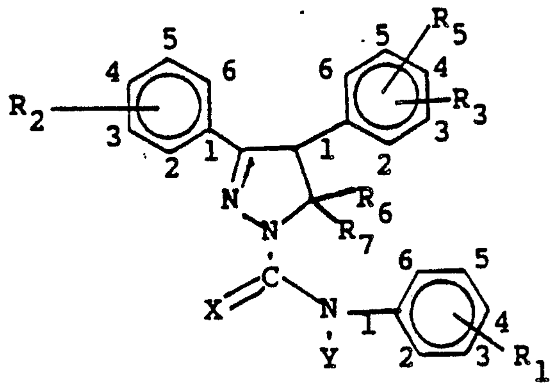

| CMPD | $R_1$ | $R_2$ | $R_3$ | $R_5$ |
|---|---|---|---|---|
| 1 | 4-$CF_3$ | 4-Cl | H | 4-$CO_2$Me |
| 2 | 4-Cl | 4-Cl | H | 4-$CO_2$Me |
| 3 | 4-$CF_3$ | 4-F | H | 4-$CO_2$Me |
| 4 | 4-Cl | 4-F | H | 4-$CO_2$Me |
| 5 | 4-$CF_3$ | 4-$CF_3$ | H | 4-$SO_2NMe_2$ |
| 6 | 4-Cl | 4-$CF_3$ | H | 4-$SO_2NMe_2$ |
| 7 | 4-SMe | 4-$CF_3$ | H | 4-$SO_2NMe_2$ |
| 8 | 4-$CF_3$ | H | H | 4-$CO_2$Me |
| 9 | 4-Cl | H | H | 4-$CO_2$Me |
| 10 | 4-Br | H | H | 4-$CO_2$Me |
| 11 | 4-Br | 4-Cl | H | 4-$CO_2$Me |
| 12 | 4-SMe | 4-Cl | H | 4-$CO_2$Me |
| 13 | 4-$CF_3$ | 4-Cl | H | 4-$CO_2$Et |
| 14 | 4-Cl | 4-Cl | H | 4-$CO_2$Et |
| 15 | 4-Br | 4-Cl | H | 4-$CO_2$Et |
| 16 | 4-SMe | 4-Cl | H | 4-$CO_2$Et |
| 17 | 4-$CF_3$ | 4-Cl | H | 4-$CO_2$-i-Pr |
| 18 | 4-Cl | 4-Cl | H | 4-$CO_2$-i-Pr |
| 19 | 4-Br | 4-Cl | H | 4-$CO_2$-i-Pr |
| 20 | 4-SMe | 4-Cl | H | 4-$CO_2$-i-Pr |

TABLE 4 (continued)

| CMPD | $R_1$ | $R_2$ | $R_3$ | $R_5$ |
|---|---|---|---|---|
| 21 | 4-$CF_3$ | 4-$CF_3$ | H | 4-$CO_2Me$ |
| 22 | 4-$CF_3$ | 4-Cl | H | 4-$CO_2Me$ |
| 23 | 4-$CF_3$ | 4-Cl | H | 4-$CONMe_2$ |
| 24 | 4-Cl | 4-Cl | H | 4-$CONMe_2$ |
| 25 | 4-Br | 4-Cl | H | 4-$CONMe_2$ |
| 26 | 4-$CF_3$ | 4-Cl | H | 4-CONHMe |
| 27 | 4-Cl | 4-Cl | H | 4-CONHMe |
| 28 | 3,4-di-Cl | 4-Cl | H | 4-CONHMe |
| 29 | 4-F | 4-Cl | H | 4-CONHMe |
| 30 | 4-$CF_3$ | 4-CN | H | 4-$CO_2Me$ |
| 31 | 4-Cl | 4-CN | H | 4-$CO_2Me$ |
| 32 | 3,4-di-Cl | 4-CN | H | 4-$CO_2Me$ |
| 33 | 4-$CF_3$ | 3-Cl | H | 4-$CO_2Me$ |
| 34 | 4-Cl | 3-Cl | H | 4-$CO_2Me$ |
| 35 | 3,4-di-Cl | 3-Cl | H | 4-$CO_2Me$ |
| 36 | 4-$CF_3$ | 4-$CO_2Me$ | H | 4-$CO_2Me$ |
| 37 | 4-Cl | 4-$CO_2Me$ | H | 4-$CO_2Me$ |
| 38 | 4-$CO_2Et$ | 4-$CO_2Me$ | H | 4-$CO_2Me$ |
| 39 | 4-$CF_3$ | 4-$OCH_2C(Cl)=CH_2$ | H | 4-$CO_2Me$ |
| 40 | 4-$CF_3$ | 4-OPh | H | 4-$CO_2Me$ |
| 41 | 4-Cl | 4-OPh | H | 4-$CO_2Me$ |
| 42 | 4-Br | 4-OPh | H | 4-$CO_2Me$ |
| 43 | 4-$CF_3$ | 3,4-di-Cl | H | 4-$CO_2Me$ |
| 44 | 4-Cl | 3,4-di-Cl | H | 4-$CO_2Me$ |

TABLE 4 (continued)

| CMPD | $R_1$ | $R_2$ | $R_3$ | $R_5$ |
|---|---|---|---|---|
| 45 | 4-Br | 3,4-di-Cl | H | 4-$CO_2$Me |
| 46 | 3,4-di-Cl | 3,4-di-Cl | H | 4-$CO_2$Me |
| 47 | 3-$CF_3$ | 4-$CO_2$Me | H | 4-$CO_2$Me |
| 48 | 4-OMe | 4-$CO_2$Me | H | 4-$CO_2$Me |
| 49 | 4-Cl | 4-Br | H | 4-$CO_2$Me |
| 50 | 4-Br | 4-Br | H | 4-$CO_2$Me |
| 51 | 4-OMe | 4-Br | H | 4-$CO_2$Me |
| 52 | 4-$CF_3$ | 4-Br | H | 4-$CO_2$Me |
| 53 | 4-$CF_3$ | 4-Cl | H | 3-$CO_2$Me |
| 54 | 4-Cl | 4-Cl | H | 3-$CO_2$Me |
| 55 | 4-Br | 4-Cl | H | 3-$CO_2$Me |
| 56 | 4-$CO_2$Et | 4-Cl | H | 3-$CO_2$Me |
| 57 | 4-$CF_3$ | 4-Cl | H | 4-$CO_2CH_2CF_3$ |
| 58 | 4-Cl | 4-Cl | H | 4-$CO_2CH_2CF_3$ |
| 59 | 4-Br | 4-Cl | H | 4-$CO_2CH_2CF_3$ |
| 60 | 4-$CF_3$ | 4-Cl | H | 4-$SO_2$NHMe |
| 61 | 4-Cl | 4-Cl | H | 4-$SO_2$NHMe |
| 62 | 4-$CF_3$ | 4-Cl | H | 4-$SO_2NMe_2$ |
| 63 | 4-Cl | 4-Cl | H | 4-$SO_2NMe_2$ |
| 64 | 4-$CF_3$ | 4-Cl | H | 4-C(O)Me |
| 65 | 4-Cl | 4-Cl | H | 4-C(O)Me |
| 66 | 4-$CF_3$ | 4-$OCF_3$ | H | 4-$CO_2$Me |
| 67 | 4-Cl | 4-$OCF_3$ | H | 4-$CO_2$Me |
| 68 | 4-Br | 4-$OCF_3$ | H | 4-$CO_2$Me |
| 69 | 4-$CF_3$ | 4-SMe | H | 4-$CO_2$Me |
| 70 | 4-Cl | 4-SMe | H | 4-$CO_2$Me |
| 71 | 4-Br | 4-SMe | H | 4-$CO_2$Me |
| 72 | 4-SMe | 4-SMe | H | 4-$CO_2$Me |

TABLE 4 (continued)

| CMPD | $R_1$ | $R_2$ | $R_3$ | $R_5$ |
|---|---|---|---|---|
| 73 | 3-Cl-4-F | 4-SMe | H | 4-$CO_2$Me |
| 74 | 4-$CF_3$ | 4-$SO_2$Me | H | 4-$CO_2$Me |
| 75 | 4-Cl | 4-$SO_2$Me | H | 4-$CO_2$Me |
| 76 | 4-Br | 4-$SO_2$Me | H | 4-$CO_2$Me |
| 77 | 4-$CF_3$ | 4-$NMe_2$ | H | 4-$CO_2$Me |
| 78 | 4-Cl | 4-$NMe_2$ | H | 4-$CO_2$Me |
| 79 | 4-Br | 4-$NMe_2$ | H | 4-$CO_2$Me |
| 80 | 3-Cl-4-F | 4-$NMe_2$ | H | 4-$CO_2$Me |
| 81 | 4-$CF_3$ | 4-Cl | H | 4-$CO_2$Me |
| 82 | 4-$CO_2$Et | 4-Cl | H | 4-$CO_2$Me |
| 83 | 3-$NO_2$-4-Cl | 4-Cl | H | 4-$CO_2$Me |
| 84 | 2-OMe-3-Cl | 4-Cl | H | 4-$CO_2$Me |
| 85 | 3-Cl-4-F | 4-Cl | H | 4-$CO_2$Me |
| 86 | 4-Cl-3-$CF_3$ | 4-Cl | H | 4-$CO_2$Me |
| 87 | 3,4-di-Cl | 4-Cl | H | 4-$CO_2$Me |
| 88 | 4-$CF_3$ | 3-Cl | H | 4-$CO_2CH_2C{\equiv}CH$ |
| 89 | 4-Cl | 4-Cl | H | 4-$CO_2CH_2C{\equiv}CH$ |
| 90 | 4-Br | 4-Cl | H | 4-$CO_2CH_2C{\equiv}CH$ |
| 91 | 4-$CF_3$ | 4-$CF_2$HO | H | 4-$CO_2$Me |
| 92 | 4-$CF_3$ | 3,4-di-F | H | 4-$CO_2$Me |
| 93 | 4-Cl | 3,4-di-F | H | 4-$CO_2$Me |
| 94 | 4-Br | 3,4-di-F | H | 4-$CO_2$Me |
| 95 | 4-$CO_2$Et | 3,4-di-F | H | 4-$CO_2$Me |
| 96 | 3-$CF_3$-4-Cl | 3,4-di-F | H | 4-$CO_2$Me |
| 97 | 4-$CF_3$ | 4-Cl | 4-Cl | 3-$CO_2$Me |
| 98 | 4-Cl | 4-Cl | 4-Cl | 3-$CO_2$Me |
| 99 | 4-Br | 4-Cl | 4-Cl | 3-$CO_2$Me |
| 100 | 4-F | 4-Cl | 4-Cl | 3-$CO_2$Me |

TABLE 4 (continued)

| CMPD | $R_1$ | $R_2$ | $R_3$ | R |
|---|---|---|---|---|
| 101 | 3-$CF_3$-4-Cl | 4-Cl | 4-Cl | 3-$CO_2$Me |
| 102 | 3-$CF_3$-4-Cl | 4-Cl | H | 4-$CO_2$Et |
| 103 | 3-Cl-4-F | 4-Cl | H | 4-$CO_2$Et |
| 104 | 4-$CF_3$ | 4-Cl | H | 4-$CO_2$Me |
| 105 | 4-Cl | 4-Cl | H | 4-$CO_2$Me |
| 106 | 4-Br | 4-Cl | H | 4-$CO_2$Me |
| 107 | 4-Cl | 4-OH | H | 4-$CO_2$Me |
| 108 | 4-Br | 4-OH | H | 4-$CO_2$Me |
| 109 | 4-$CF_3$ | 4-OH | H | 4-$CO_2$Me |
| 110 | 4-$CF_3$ | 4-OMe | H | 4-$CO_2$Me |
| 111 | 4-Br | 4-OMe | H | 4-$CO_2$Me |
| 112 | 4-$CF_3$ | 4-Cl | H | 4-$CO_2$Et |
| 113 | 4-$CF_3$ | 4-$CO_2$Me | H | 4-$CO_2$Me |
| 114 | 4-$CF_3$ | 4-Br | H | 4-$CO_2$Me |
| 115 | 4-$CF_3$ | 4-Cl | H | 4-$CO_2$Me |

## Example 10

### Fall Armyworm

A test unit consisted of an 8-ounce plastic cup containing a layer of wheat germ diet, approximately 0.5 cm thick infested with ten third-instar larvae of fall armyworm (*Spodoptera frugiperda*). Solutions of each of the above-listed test compounds were made by combining the compound in an acetone/distilled water 75/25 solvent and then sprayed onto the cups; a single solution per cup replicated three times. Spraying was accomplished by passing the cups, on a conveyor belt, directly beneath a flat fan hydraulic nozzle which discharged the spray at a rate of 0.5 pounds of active ingredient per acre (about 0.55 kg/ha) at 30 p.s.i. The cups were then covered and held at 27°C and 50% relative humidity for 72 hours, after which time mortality readings were taken. Tested compounds which killed 80% or more of the larvae are listed below:

| | | | | | |
|---|---|---|---|---|---|
| 1 | 16 | 30 | 46 | 59 | 86 |
| 2 | 17 | 31 | 49 | 60 | 91 |
| 3 | 18 | 32 | 50 | 62 | 92 |
| 4 | 19 | 33 | 52 | 66 | 93 |
| 8 | 21 | 34 | 53 | 67 | 104 |
| 10 | 22 | 41 | 54 | 68 | 105 |
| 11 | 23 | 42 | 55 | 79 | 106 |
| 13 | 26 | 43 | 56 | 81 | 114 |
| 14 | 27 | 44 | 57 | 82 | 115 |
| 15 | 28 | 45 | 58 | 85 | |

## Example 11

### Tobacco Budworm

The test procedure of Example 10 was repeated for efficacy against third-instar larvae of the tobacco budworm (Heliothis virescens) except that mortality was assessed at 48 hours. Tested compounds which killed 80% or more of the larvae are listed below:

| | | | | |
|---|---|---|---|---|
| 1 | 17 | 32 | 58 | 104 |
| 2 | 18 | 33 | 59 | 106 |
| 3 | 19 | 44 | 62 | 112 |
| 4 | 21 | 45 | 66 | 114 |
| 8 | 22 | 46 | 67 | 115 |
| 11 | 23 | 49 | 81 | |
| 13 | 26 | 50 | 85 | |
| 14 | 28 | 52 | 86 | |
| 15 | 30 | 53 | 91 | |
| 16 | 31 | 57 | 92 | |

## Example 12

### European Corn Borer

A test unit consisted of an 8-ounce plastic cup containing a one-inch square of wheat germ/soyflour diet infested with five third-instar larvae of the European corn borer (Ostrinia nubilalis). Test units were sprayed as described in Example 10. The cups were then covered and held at 27° C and 50% relative himidity for 48 hours, after which time mortality readings were taken. Tested compounds which killed 80% or more of the larvae are listed below:

| | |
|---|---|
| 1 | 21 |
| 2 | 22 |
| 3 | 26 |
| 4 | 30 |

| | |
|---|---|
| 11 | 33 |
| 12 | 50 |
| 13 | 52 |
| 14 | |
| 15 | |

Example 13

Southern Corn Rootworm

A test unit consisted of an 8-ounce plastic cup containing 1 sprouted corn seed. The test units were sprayed as described in Example 10. After the treated cup had dried it was infested with five third-instar larvae of the Southern corn rootworm (Diabrotica undecimpunctata howardi) and replicated three times. A moistened dental wick was inserted into each cup to prevent drying and the cups were covered. The cups were then held at 27° C and 50% humidity for 48 hours, after which time mortality readings were taken. Tested compounds which killed 80% or more of the larvae are listed below:

8
10
28
55
56
58
91
93

Example 14

Boll Weevil

The test unit consisted of five adult boll weevils (Anthonomus grandis) in a 9-ounce cup. The test procedure employed was the same as in Example 10. Mortality readings were taken 48 hours after treatment. Tested compounds which killed 80% or more of the adults are listed below:

| | |
|---|---|
| 1 | 15 |
| 2 | 21 |
| 3 | 22 |
| 4 | 26 |
| 12 | 30 |
| 13 | 33 |
| 14 | 52 |

Example 15

Aster Leafhopper

A test unit was a 12-ounce cup containing oat (Avena sativa) seedlings in a 1-inch layer of sterilized soil. Test units were sprayed as described in Example 10. After the treated oats had dried between 10 and 15 adult aster leafhoppers (Mascrosteles fascifrons) were aspirated into each of the covered cups. The cups were held at 27° C and 50% relative humidity for 48 hours, and evaluated for mortality. Tested compounds which killed 80% or more of the insects are 22, 49, 50, 58 and 91.

## Claims

1. A compound of the formula

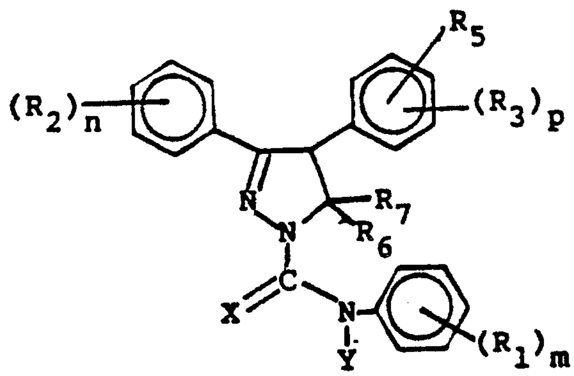

wherein
$R_1$, $R_2$ and $R_3$ are independently selected from $R_8$, halogen, CN, $N_3$, SCN, $NO_2$, $OR_8$, $SR_8$, $S(O)R_8$, $S(O)_2R_8$, $OC(O)R_8$, $OS(O)_2R_8$, $C(O)OR_8$, $C(O)R_8$, $C(O)NR_8R_9$, $S(O)_2NR_8R_9$, $NR_8R_9$, $NR_9C(O)R_8$, $OC(O)NHR_8$, $NR_9C(O)NHR_8$ and $NR_9S(O)_2R_8$, or when m, n or p is 2, $R_1$, $R_2$ or $R_3$ can be taken together as $OCH_2O$, $OCH_2CH_2O$ or $CH_2CH_2O$, to form a 5 or 6 membered ring, each of which can be substituted with 1 to 4 halogen atoms or 1 to 2 methyl groups;
$R_5$ is selected from $C(O)OR_{10}$, $C(O)R_{10}$, $C(O)NR_{10}R_{11}$, $C(S)SR_{10}$, $C(S)NR_{10}R_{11}$ and $S(O)_2NR_{10}R_{11}$;
$R_6$ is H or $C_1$ to $C_4$ alkyl;
$R_7$ is H or $CH_3$;
$R_8$ and $R_{10}$ are independently selected from H, $C_1$ to $C_4$ alkyl, $C_3$ to $C_4$ alkenyl, $C_3$ to $C_4$ alkynyl, $C_1$ to $C_4$ haloalkyl, $C_3$ to $C_4$ haloalkenyl, $C_1$ to $C_4$ alkyl substituted with CN, $C(O)OCH_3$, $C(O)OCH_2CH_3$, $OCH_3$, $OCH_2CH_3$, $SCH_3$,$SCH_2CH_3$ and $NO_2$, and phenyl or benzyl, either optionally substituted with W, or $R_8$ and $R_9$ can be taken together as $(CH_2)4$, $(CH_2)_5$ or $CH_2CH_2OCH_2CH_2$;
$R_9$ and $R_{11}$ are independently selected from H, $C_1$ to $C_4$ alkyl, $C_3$ to $C_4$ alkenyl, $C_3$ to $C_4$ alkynyl, and $C_1$ to $C_4$ haloalkyl, or $R_{10}$ and $R_{11}$ can be taken together as $(CH_2)_4$, $(CH_2)_5$ or $CH_2CH_2OCH_2CH_2$;
m and n are independently 0 to 5;
p is 0 to 4;
W is selected from halogen, CN, $NO_2$, $C_1$ to $C_2$ alkyl, $C_1$ to $C_2$ haloalkyl, $C_1$ to $C_2$ alkoxy, $C_1$-$C_2$ haloalkoxy, $C_1$ to $C_2$ alkylthio, $C_1$ to $C_2$ haloalkylthio, $C_1$ to $C_2$ alkylsulfonyl and $C_1$ to $C_2$ haloalkylsulfonyl;
X is O or S; and
Y is selected from H, $C_1$ to $C_6$ alkyl, $C_2$ to $C_6$ alkoxyalkyl, CHO, $C_2$ to $C_6$ alkylcarbonyl, $C_2$ to $C_6$ alkoxycarbonyl, $C_2$ to $C_6$ haloalkylcarbonyl, $C_1$ to $C_6$ alkylthio, $C_1$ to $C_6$ haloalkylthio, phenylthio, and phenylthio substituted with 1 to 3 substituents independently selected from W.

2. A compound according to Claim 1 wherein:
$R_6$ is H;
$R_7$ is H;
n and p are independently 0 to 2; and
m is 1 to 2.

3. A compound according to Claim 2 wherein:
$R_1$, $R_2$, and $R_3$ are independently $R_8$, halogen, CN, $NO_2$, $OR_8$, $SR_8$, $S(O)R_8$, $S(O)_2R_8$ or $NR_8R_9$, or when m, n or p is 2, $R_1$, $R_2$ or $R_3$ can be taken together as $OCH_2O$, $OCH_2CH_2O$ or $CH_2CH_2O$, each of which can be substituted with 1 to 4 halogen atoms or 1 to 2 methyl groups;
$R_8$ is $C_1$ to $C_2$ alkyl, $C_3$ to $C_4$ alkenyl, $C_1$ to $C_2$ haloalkyl, $C_3$ to $C_4$ haloalkenyl or phenyl optionally substituted with halogen;
$R_9$ is H or $C_1$ to $C_2$ alkyl; and
X is O.

4. A compound according to Claim 3 wherein:
$R_1$ is halogen, CN, $NO_2$, $OCF_2H$, $OCF_3$ $OCF_2CF_2H$, $CF_3$ or when m is 2 then $R_1$ may be taken together as $CH_2C(CH_3)_2O$ or $CF_2CF_2O$ to form a 5 membered ring.
$R_2$ is H, halogen, CN, $NO_2$, $OCH_3$, $OCF_2H$, $OCF_3$, $SCH_3$, $SCF_2H$, $SCF_3$, $CF_3$, $OCF_2CF_2H$ or phenoxy;
$R_3$ is halogen;
$R_5$ is $C(O)OR_{10}$, $C(O)R_{10}$, $C(O)NR_{10}R_{11}$ or $S(O)_2NR_{10}R_{11}$;
$R_{10}$ is H, $C_1$ to $C_4$ alkyl, $C_3$ to $C_4$ alkenyl, $C_3$ to $C_4$ alkynyl, $C_1$ to $C_4$ haloalkyl, $C_3$ to $C_4$ haloalkenyl, $C_1$ to $C_4$ alkyl substituted with CN, $C(O)OCH_3$, $C(O)OCH_2CH_3$, $OCH_3$, $OCH_2CH_3$, $SCH_3$, $SCH_2CH_3$ and $NO_2$;
$R_{11}$ is H or $C_1$ to $C_2$ alkyl; and
Y is H, $C_1$ to $C_6$ alkyl, CHO, $C_2$ to $C_6$ alkylcarbonyl or $C_2$ to $C_6$ alkoxycarbonyl.

5. A compound according to Claim 4 wherein:
$R_5$ is $C(O)OR_{10}$;
$R_{10}$ is $C_1$ to $C_3$ alkyl, $C_2$ to $C_3$ haloalkyl, propargyl or allyl; and one or $R_3$ or $R_5$ is in the para-position and one of $R_1$ is in the para-position; and
Y is H, $CH_3$ $C(O)CH_3$, $C(O)OCH_3$ or CHO.

6. A compound according to Claim 5 which is: methyl 4-[3-(4-chlorophenyl)-4,5-dihydro-1-[[4-(trifluoromethyl)phenylamino]carbonyl]-1H-pyrazol-4-yl]benzoate.

7. A compound according to Claim 5 which is: methyl 4-[3(4-chlorophenyl)-1-[(4-chlorophenyl)-aminocarbonyl]-4,5-dihydro-1H-pyrazol-4-yl]benzoate.

8. A compound according to Claim 5 which is: methyl 4-[3-(4-fluorophenyl)-4,5-dihydro-1-[[4-(trifluoromethyl)phenylamino]carbonyl]-1H-pyrazol-4-yl]benzoate.

9. A compound according to Claim 5 which is: methyl 4-[1-(4-chlorophenyl)aminocarbonyl]-[3-(4-fluorophenyl)-4,5-dihydro-1H-pyrazol-4-yl]benzoate.

10. A compound according to Claim 5 which is: ethyl 4-[3-(4-chlorophenyl)-4,5-dihydro-1-[[4-(trifluoromethyl)phenylamino]carbonyl]-1H-pyrazol-4-yl]benzoate.

11. A compound according to Claim 5 which is: ethyl 4-[1-[(4-bromophenyl)aminocarbonyl]-3-(4-chlorophenyl)-4,5-dihydro-1H-pyrazol-4-yl]benzoate.

12. A compound according to Claim 5 which is: methyl, 4-[3-(4-bromophenyl)-4,5-dihydro-1-[[4-(trifluoromethyl)phenylamino]carbonyl]-1H-pyrazol-4-yl]benzoate.

13. A compound according to Claim 5 which is: methyl 4-[3-(4-cyanophenyl)-4,5-dihydro-1-[[4-(trifluoromethyl)phenylamino]carbonyl]-1H-pyrazol-4-yl]benzoate.

14. A composition comprising an insecticidally effective amount of a compound according to any one of Claims 1 to 13 together with a carrier therefor.

15. A method for controlling insects comprising applying to the insect or its environment an insecticidally effective amount of a compound according to any one of Claims 1 to 13.

European Patent Office

# EUROPEAN SEARCH REPORT

Application Number

EP 88 30 6478

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | EP-A-0 153 127 (ROHM AND HAAS CO.) * page 2, line 13 - page 6a, line 4, in particular page 3, lines 16-20 and page 4, lines 7-12; page 134, line 1 - page 135, line 23 * | 1,14,15 | C 07 D 231/06<br>C 07 D 405/04<br>C 07 D 405/12<br>A 01 N 43/56 //<br>C 07 F 7/18<br>C 07 C 69/76 |
| A,D | EP-A-0 021 506 (DUPHAR INTERNATIONALE RESEARCH B.V.) * claims 1,5,10 * | 1,14,15 | |
| A | DE-A-2 529 689 (N.V. PHILIPS GLOEILAMPENFABRIEK) * claims 1,19,35; & US - A - 4 156 007 (cat. D) * | 1,14,15 | |
| A | GB-A-2 166 137 (F.M.C. CORPORATION) * claims 1,27,28 * | 1,14,15 | |
| | | | **TECHNICAL FIELDS SEARCHED (Int. Cl.4)** |
| | | | C 07 D 231/00<br>C 07 D 405/00<br>A 01 N 43/00 |

The present search report has been drawn up for all claims

| Place of search | Date of completion of the search | Examiner |
|---|---|---|
| BERLIN | 21-10-1988 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document